(19) Europäisches Patentamt
European Patent Office
Office européen des brevets

(11) Veröffentlichungsnummer: **0 126 401**
**A1**

(12) # EUROPÄISCHE PATENTANMELDUNG

(21) Anmeldenummer: **84105449.7**

(22) Anmeldetag: **14.05.84**

(51) Int. Cl.³: **C 07 D 401/04**, A 61 K 31/405

(30) Priorität: **17.05.83 US 495370**

(43) Veröffentlichungstag der Anmeldung: **28.11.84**
**Patentblatt 84/48**

(84) Benannte Vertragsstaaten: **AT BE CH DE FR GB IT LI LU NL SE**

(71) Anmelder: **CIBA-GEIGY AG, Postfach, CH-4002 Basel (CH)**

(72) Erfinder: **Browne, Leslie J., Dr., 41 Malapardis Road, Morris Plains New Jersey 07950 (US)**

(74) Vertreter: **Zumstein, Fritz sen., Dr. et al, Bräuhausstrasse 4, D-8000 München 2 (DE)**

(54) **Neue Indolverbindungen.**

(57) N-Pyridylindole der Formel I

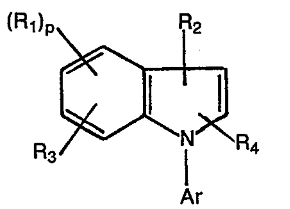

(I),

worin Ar für unsubstituiertes oder durch Niederalkyl substituiertes 3- oder 4-Pyridyl steht, die Reste $R_1$ unabhängig voneinander Wasserstoff, Halogen, Trifluormethyl, Niederalkyl, Hydroxy, acyliertes oder veräthertes Hydroxy oder Niederalkyl-(thio, -sulfinyl oder -sulfonyl) bedeuten, oder zwei an benachbarten Kohlenstoffatomen sitzende Reste $R_1$ Alkylendioxy darstellen, p für 1 oder 2 steht, $R_2$ Wasserstoff oder Niederalkyl bedeutet, einer der Reste $R_3$ und $R_4$ für Wasserstoff steht und der jeweils andere die Gruppe A–B bedeutet, in welcher
A für Alkylen mit 1 bis 12 Kohlenstoffatomen, Alkinylen oder Alkenylen mit jeweils 2 bis 12 Kohlenstoffatomen, Niederalkylenphenylen, Niederalkylen-(thio oder oxy)-niederalkylen, Niederalkylen-(thio oder oxy)-phenylen, Niederalkylenphenylen-nieder-(alkylen oder alkenylen) oder Alkadienylen mit 4 bis 12 Kohlenstoffatomen steht, und
B Carboxy, verestertes Carboxy, Carbamoyl, Mono- oder Diniederalkylcarbamoyl, Cyan, Hydroxycarbamoyl, 5-Tetrazolyl, unsubstituiertes oder durch Niederalkyl substituiertes 4,5-Dihydro-2-oxazolyl bedeutet, ihre Pyridyl-N-oxide, und ihre Salze hemmen die Thromboxan-Synthetase. Sie werden nach an sich bekannten Verfahren hergestellt.

EP 0 126 401 A1

CIBA-GEIGY AG
Basel (Schweiz)

4-14437/+

Neue Indolverbindungen

Die Erfindung betrifft neue N-Pyridylindole und Verfahren zu ihrer Herstellung.

Die Erfindung betrifft insbesondere N-Pyridylindole der Formel I

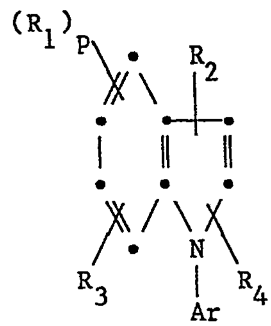

(I) ,

worin Ar für unsubstituiertes oder durch Niederalkyl substituiertes 3- oder 4-Pyridyl steht, die Reste $R_1$ unabhängig voneinander Wasserstoff, Halogen, Trifluormethyl, Niederalkyl, Hydroxy, acyliertes oder veräthertes Hydroxy oder Niederalkyl-(thio, -sulfinyl oder -sulfonyl) bedeuten, oder zwei an benachbarten Kohlenstoffatomen sitzende Reste $R_1$ Alkylendioxy darstellen, p für 1 oder 2 steht, $R_2$ Wasserstoff oder Niederalkyl bedeutet, einer der Reste $R_3$ und $R_4$ für Wasserstoff steht und der jeweils andere die Gruppe A-B bedeutet, in welcher

A für Alkylen mit 1 bis 12 Kohlenstoffatomen, Alkinylen oder Alkenylen mit jeweils 2 bis 12 Kohlenstoffatomen, Niederalkylenphenylen, Niederalkylen-(thio oder oxy)-niederalkylen, Niederalkylen-(thio oder oxy)-phenylen, Niederalkylenphenylen-nieder-(alkylen oder alkenylen) oder Alkadienylen mit 4 bis 12 Kohlenstoffatomen steht, und

B Carboxy, verestertes Carboxy, Carbamoyl, Mono- oder Di-niederalkylcarbamoyl, Cyan, Hydroxycarbamoyl, 5-Tetrazolyl, unsubstituiertes oder durch Niederalkyl substituiertes 4,5-Dihydro-2-oxazolyl bedeutet, ihre Pyridyl-N-oxide, und ihre Salze, insbesondere ihre pharmazeutisch verwendbaren Salze, sowie Verfahren zu ihrer Herstellung, pharmazeutische Präparate enthaltend diese Verbindungen und ihre Verwendung zur Herstellung pharmazeutischer Präparate oder als pharmakologisch wirksame Verbindungen.

Die vor-, sowie nachstehend verwendeten Allgemeinbegriffe haben im Rahmen der vorliegenden Erfindung die folgenden Bedeutungen:

Ein Alkylenrest bedeutet Alkylen mit 1 bis 12 Kohlenstoffatomen, das geradkettig oder verzweigt sein kann, und vorzugsweise für Propylen, Butylen, Pentylen, Hexylen oder Heptylen steht, wobei die genannten Reste unsubstituiert oder durch eine oder mehrere Niederalkylgruppen substituiert sind, mit der Massgabe, dass die Summe der Kohlenstoffatome nicht grösser als 12 ist.

Der Ausdruck Alkenylen bedeutet einen Alkenylenrest mit 2 bis 12 Kohlenstoffatomen, der geradkettig oder verzweigt sein kann, und vorzugsweise für Propenylen, 1- oder 2-Butenylen, 1- oder 2-Pentenylen, 1-, 2- oder 3-Hexenylen oder 1-, 2-, 3- oder 4-Heptenylen steht. Die genannten Reste sind unsubstituiert oder durch eine oder mehrere Niederalkylgruppen substituiert, mit der Massgabe, dass die Summe der Kohlenstoffatome 12 nicht übersteigt.

Der Begriff Alkinylen bezeichnet einen Alkinylenrest mit 2 bis 12 Kohlenstoffatomen, der geradkettig oder verzweigt sein kann, und vorzugsweise für Propinylen, 1- oder 2-Butinylen, 1- oder 2-Pentinylen, 1-, 2- oder 3-Hexinylen oder 1-, 2-, 3- oder 4-Heptinylen steht. Diese Reste sind unsubstituiert oder durch eine oder mehrere Niederalkylgruppen substituiert, wobei die Summe der Kohlenstoffatome 12 nicht übersteigt.

Alkadienylen bedeutet einen konjugierten oder unkonjungierten Alkadienylenrest mit 4 bis 12 Kohlenstoffatomen, der geradkettig oder verzweigt sein kann, und z.B. für Butadienylen, 1,3- oder 1,4-Pentadienylen, 1,3-, 1,4-, oder 1,5-Hexadienylen oder 1,3-, 1,4-, 1,5- oder 1,6-Heptadienylen steht. Die genannten Reste können unsubstituiert oder durch eine oder mehrere Niederalkylgruppen substituiert sein, mit der Massgabe, dass die Summe der Kohlenstoffatome nicht grösser als 12 ist.

Der Ausdruck Phenylen bedeutet 1,2-, 1,3- und vorzugsweise 1,4-Phenylen.

Der Ausdruck "nieder" umfasst in den vor- oder nachstehend genannten organischen Gruppen, Resten oder Verbindungen insbesondere solche mit höchstens 7, vorzugsweise 4 und vor allem 1, 2 oder 3 Kohlenstoffatomen.

Niederalkylen-phenylen, Niederalkylen-phenylen-nieder-(alkylen oder alkenylen) oder Niederalkylen-(thio oder oxy)-phenylen enthalten in jedem Alkylenrest vorzugsweise 1 bis 4 und insbesondere 1 oder 2 Kohlenstoffatome, und in jedem Alkenylenrest vorzugsweise 2 bis 4 Kohlenstoffatome. Die Niederalkylen- oder Niederalkenylenreste können geradkettig oder verzweigt sein.

Eine Niederalkylen-(thio oder oxy)-niederalkylengruppe kann geradkettig oder verzweigt sein und insgesamt 2 bis 12, vorzugsweise 2 bis 8, Kohlenstoffatome haben.

Niederalkyl enthält vorzugsweise 1 bis 4 Kohlenstoffatome und steht z.B für Aethyl, Propyl oder Butyl und insbesondere für Methyl.

Niederalkylen enthält vorzugsweise 1 bis 4 Kohlenstoffatome und steht z.B für Methylen, Aethylen, 1,2- oder 1,3-Propylen oder 1,2-, 1,3- oder 1,4-Butylen.

Niederalkylendioxy bedeutet vorzugsweise Aethylendioxy oder Methylendioxy.

Niederalkoxy enthält vorzugsweise 1 bis 4 Kohlenstoffatome und ist z.B. Aethoxy, Propoxy oder insbesondere Methoxy. Eine Niederalkyl-(thio, -sulfinyl oder -sulfonyl)-Gruppe ist vorzugsweise Methylthio, Methylsulfinyl bzw. Methylsulfonyl.

Niederalkoxycarbonyl enthält vorzugsweise 1 bis 4 Kohlenstoffatome im Alkoxyteil und bedeutet z.B. Methoxycarbonyl, Propoxycarbonyl oder Isopropoxycarbonyl und insbesondere Aethoxycarbonyl. Mono-(niederalkyl)-carbamoyl hat vorzugsweise 1 bis 4 Kohlenstoffatome im Alkylteil und ist z.B. N-Methylcarbamoyl, N-Propylcarbamoyl oder insbesondere N-Aethylcarbamoyl. Di-(niederalkyl)-carbamoyl enthält vorzugsweise 1 bis 4 Kohlenstoffatome in jeder Niederalkylgruppe und steht z.B. für N,N-Dimethylcarbamoyl, N-Methyl-N-äthylcarbamoyl und insbesondere für N,N-Diäthylcarbamoyl.

Halogen ist vorzugsweise Fluor oder Chlor, kann aber auch Brom oder Jod sein.

Aryl, z.B. in einer Aryl-niederalkoxy-Gruppe, bedeutet vorzugsweise Phenyl, durch Niederalkyl, Halogen oder Niederalkoxy mono- oder disubstituiertes Phenyl oder Pyridyl.

Aryl-niederalkoxy ist vorzugsweise Benzyloxy.

Acyliertes Hydroxy bedeutet vorzugsweise Niederalkanoyloxy, z.B. Acetyloxy, unsubstituiertes oder im Phenylring durch Niederalkyl, z.B. Methyl, Halogen, z.B. Chlor, oder Niederalkoxy, z.B. Methoxy, substituiertes Benzoyloxy oder Nikotinoyloxy.

Veräthertes Hydroxy bedeutet vorzugsweise Niederalkoxy, z.B. Methoxy, unsubstituiertes oder im Phenylring durch Niederalkyl, z.B. Methyl, Halogen, z.B. Chlor, oder Niederalkoxy, z.B. Methoxy, substituiertes Benzyloxy oder Pyridylmethoxy.

Unter verestertem Carboxy sind vorzugsweise solche Ester zu verstehen, die pharmazeutisch verwendbar sind, insbesondere solche, die durch Solvolyse oder unter physiologischen Bedingungen in die entsprechenden freien Säuren überführbar sind, z.B. Niederalkoxycarbonyl, (Amino-, Mono- oder Di-niederalkylamino)-substituiertes Niederalkoxycarbonyl, Carboxy-substituiertes Niederalkoxycarbonyl, z.B. α-Carboxy-substituiertes Niederalkoxycarbonyl, Niederalkoxycarbonyl-substituiertes Niederalkoxycarbonyl, z.B. α-Niederalkoxycarbonyl-substituiertes Niederalkoxycarbonyl, Aryl-substituiertes Niederalkoxycarbonyl, z.B. gegebenenfalls substituiertes Benzyloxycarbonyl oder Pyridylmethoxycarbonyl, (Hydroxy-, Niederalkanoyloxy- oder Niederalkoxy)-substituiertes Niederalkoxycarbonyl, z.B. Pivaloyloxymethoxycarbonyl, (Hydroxy-, Niederalkanoyloxy- oder Niederalkoxy)-substituiertes Niederalkoxymethoxycarbonyl, Bicycloalkoxycarbonyl-substituiertes Niederalkoxycarbonyl, z.B. Bicyclo[2,2,1]-heptyloxycarbonyl-substituiertes Niederalkoxycarbonyl, insbesondere Bicyclo[2,2,1]-heptyloxycarbonyl-substituiertes Methoxy, z.B. Bornyloxycarbonylmethoxycarbonyl, 3-Phthalidoxycarbonyl, (Niederalkyl-, Niederalkoxy-, Halogen)-substituiertes 3-Phthalidoxycarbonyl, Niederalkoxycarbonyloxy-niederalkoxycarbonyl, z.B. 1-(Methoxy- oder Aethoxycarbonyloxy)-äthoxycarbonyl, Aryloxycarbonyl, z.B. Phenoxycarbonyl oder Phenoxycarbonyl, das vorteilhaft in der ortho-Position durch Carboxy oder Niederalkoxycarbonyl substituiert ist.

Salze sind vorzugsweise pharmazeutisch verwendbare Salze, insbesondere Metall- oder Ammoniumsalze der genannten Verbindungen der Formel I, welche eine freie Carboxygruppe besitzen, vor allem Alkalimetall- oder Erdalkalimetallsalze, z.B. Natrium-, Kalium-, Magnesium- oder Calciumsalze; in erster Linie leicht kristallisierende Ammoniumsalze, die abgeleitet sind von Ammoniak oder organischen Aminen, z.B. Mono-, Di- oder Tri-nieder-(alkyl oder cycloalkyl)-aminen, Tri(hydroxyniederalkyl)-aminen, Niederalkylendiaminen oder (Hydroxy-niederalkyl- oder Aryl-niederalkyl)-niederalkylammonium-Hydroxiden, z.B. Methylamin, Diäthylamin, Triäthylamin, Dicyclohexylamin, Triäthanolamin, Aethylendiamin, Tris-(hydroxymethyl)-aminomethan oder Benzyl-trimethylammoniumhydroxid. Die genannten Verbindungen der Formel I bilden Säureadditionssalze. Diese werden vorzugsweise mit solchen anorganischen oder organischen Säuren, die pharmazeutisch verwendbare Säureadditionssalze ergeben, hergestellt. Solche Säuren sind z.B. starke Mineralsäuren, wie Halogenwasserstoffsäuren, z.B. Chlorwasserstoff- oder Bromwasserstoffsäure, Schwefel-, Phosphor-, Salpeter- oder Perchlorsäure; oder organische Säuren, insbesondere aliphatische oder aromatische Carbon- oder Sulfonsäuren, z.B. Ameisen-, Essig-, Pripion-, Bernstein-, Glykol-, Milch-, Aepfel-, Wein-, Glucon-, Zitronen-, Malein-, Fumar-, Brenztrauben-, Phenylessig-, Benzoe-, 4-Aminobenzoe-, Anthranil-, 4-Hydroxybenzoe-, Salicyl-, 4-Aminosalicyl-, Pamoe-, Nikotin-, Methansulfon-, Aethansulfon-, Hydroxyäthansulfon-, Benzolsulfon-, p-Toluolsulfon-, Naphthalinsulfon-, Sulfanil- oder Cyclohexylsulfaminsäure; oder andere saure organische Substanzen, wie z.B. die Ascorbinsäure.

Zur Isolierung oder Reinigung können auch pharmazeutisch ungeeignete Salze Verwendung finden. Zur therapeutischen Anwendung gelangen nur die pharmazeutisch verwendbaren, nicht toxischen Salze, die deshalb bevorzugt sind.

Die Verbindungen der Erfindung zeigen wertvolle pharmakologische Eigenschaften, z.B. kardiovaskuläre Effekte, indem sie die Thromboxan-Ausschüttung in Säugern selektiv hemmen. Diese Hemmung kommt durch selektive Herabsetzung des Thromboxan-Synthetase-Spiegels zustande. Die Verbindungen sind daher nützlich zur Behandlung von solchen Krankheiten bei Säugern, die auf eine Hemmung der Thromboxan-Synthetase ansprechen. Solche Krankheiten sind in erster Linie kardiovaskuläre Störungen wie Thrombose, Arteriosklerose, Koronarkrämpfe, cerebrale ischämische Anfälle, Migräne und andere vaskuläre Kopfschmerzen, Myokardinfarkt, Angina pectoris und Hypertension.

Diese Wirkungen können durch in vitro-Versuche oder in vivo-Tierversuche, vorzugsweise an Säugetieren, z.B. an Meerschweinchen, Mäusen, Ratten, Katzen, Hunden oder Affen, nachgewiesen werden. Die genannten Verbindungen können den Versuchstieren enteral oder parenteral, vorzugsweise oral oder subkutan, intravenös oder intraperitoneal, z.B. mittels Gelatine-Kapseln oder in Form von Stärke enthaltenden Suspensionen oder wässerigen Lösungen, verabreicht werden. Die verwendete Dosis kann in einem Bereich von ungefähr 0,01 bis 100 mg/kg/Tag, vorzugsweise von ungefähr 0,05 bis 50 mg/kg/Tag und insbesondere von ungefähr 0,1 bis 25 mg/kg/Tag liegen.

Die in vitro-Hemmung des Thromboxan-Synthetase-Enzyms kann analog der Methode von Sun [Biochem. Biophys. Res. Comm. 74, 1432 (1977)] nachgewiesen werden. Das Testverfahren wird wie folgt durchgeführt:

$^{14}C$-Arachidonsäure wird mit einem Enzymgemisch-Präparat, bestehend aus solubilisierter und partiell gereinigter Prostaglandin-cyclooxygenase von Schaf-Samenblasen und aus einem rohen Mikrosomen-Präparat von Thromboxan-Synthetase, das von lysierten menschlichen Blutplättchen stammt, inkubiert. Die Testverbindung (gelöst in einem Puffer, oder, falls nötig, in wenig Aethanol) wird zu dem Inkubationsmedium gegeben. Am Ende der Inkubationsperiode (30 Minuten) wird das Prostaglandin $E_2$ ($PGE_2$) durch Hinzufügen von Natriumborhydrid zu einem Gemisch aus Prostaglandin $F_2\alpha$ und $F_2ß$ [$PGF_2\alpha+ß$] reduziert. Die radioaktiven Produkte und das überschüssige Substrat werden mit Essigsäureäthylester extrahiert und der Extrakt zur Trockene eingedampft. Der Rückstand wird in Aceton gelöst, auf Dünnschichtplatten aufgetragen und mit dem Lösungsmittelsystem Toluol/Aceton/Eisessig [100:100:3 (Volumen)] chromatographiert. Die radioaktiven Zonen werden lokalisiert. Die Zonen von Thromboxan $B_2$ ($TxB_2$) und $PGF_2\alpha+ß$ werden in Szintillationsröhrchen für Flüssigkeiten übertragen und gezählt. Der Quotient aus den Zahlenwerten von $TxB_2$ und $PGF_2\alpha+ß$ wird für jede Konzentration der Testverbindung

berechnet und daraus die $IC_{50}$-Werte graphisch ermittelt. Dieser Wert entspricht der Konzentration an Testverbindung, bei welcher der Quotient $TxB_2/PGF_2\alpha+\beta$ auf 50 % des Kontrollwertes reduziert wird.

Die in vitro-Wirkung der Verbindungen auf Prostaglandin-cyclooxygenase wird gemäss einer Modifikation der Methode von Takeguchi et al.[Biochemistry 10, 2372 (1971)] gemessen. Das Testverfahren läuft wie folgt ab:

Lyophilisierte Samenblasen-Mikrosomen von Schafen werden als Prostaglandin-synthetisierendes Enzympräparat verwendet. Es wird die Umwandlung von $^{14}C$-Arachidonsäure in $PGE_2$ gemessen. Die Testverbindungen (gelöst in einem Puffer, oder, wenn nötig, in wenig Aethanol) werden zu dem Inkubationsgemisch gegeben. Die Prostaglandine werden extrahiert und durch Dünnschichtchromatographie aufgetrennt. Die Platten werden überprüft, die dem $PGE_2$ entsprechenden radioaktiven Zonen in Szintillationsröhrchen für Flüssigkeiten übertragen und ihre Radioaktivität gezählt. Zur Bestimmung der $IC_{50}$-Werte für die Hemmung wird graphisch die Konzentration an Testverbindung ermittelt, bei welcher die Menge des synthetisierten $PGE_2$ um 50 % reduziert wird.

Die in-vitro-Wirkung auf Prostacyclin-($PGI_2$)-Synthetase wird analog der Methode von Sun et al., Prostaglandins 14, 1055 (1977) bestimmt. Das Testverfahren läuft wie folgt ab:

$^{14}C$-Arachidonsäure wird mit einem Enzymgemisch inkubiert, das aus solubilisierter und partiell gereinigter Prostaglandin-cyclooxygenase von Schaf-Samenblasen sowie aus roher $PGI_2$-Synthetase besteht, die in Form einer Mikrosomen-Fraktion, gewonnen aus Rinderaorten, vorliegt.

Die Testverbindung (gelöst in einem Puffer, oder, wenn nötig, in wenig Aethanol) wird in das Inkubationsmedium gegeben. Das Reaktionsgemisch wird in 100 mMolarem Tris HCl (pH 7,5) 30 Minuten bei 37°C inkubiert, auf den pH-Wert 3 angesäuert und mit Essigsäureäthylester extrahiert. Der Extrakt wird zur Trockene eingedampft, der Rückstand in Aceton gelöst, auf Dünnschicht-Platten aufgetragen und mit dem von Sun et al. beschriebenen Lösungsmittelsystem chromatographiert. Die radioaktiven Zonen werden mit einem Detektor lokalisiert. Die dem 6-Keto-$PGF_1\alpha$ (einem stabilen Endprodukt der Prostacyclin-Biotransformation) und $PGE_2$ entsprechenden Zonen werden in Szintillationsröhrchen für Flüssigkeiten übertragen und gezählt. Der Quotient aus den Zahlenwerten von 6-Keto-$PGF_1\alpha$ und $PGE_2$ wird für jede Konzentration der verwendeten Testverbindung berechnet und daraus die $IC_{50}$-Werte der Hemmung graphisch ermittelt. Dieser Wert entspricht der Konzentration an Testverbindung, bei welcher der Quotient 6-Keto-$PGF_1\alpha/PGE_2$ auf 50 % des Kontrollwertes reduziert wird.

Die Hemmung der Thromboxan-Synthese und die Verminderung des Thromboxan-Plasmaspiegels wird in vivo durch Verabreichung der Testverbindung an Ratten [analog zu den von Tai et al. in Anual. Biochem. 87, 343 (1978) und von Salmon in Prostaglandins 15, 383 (1978) beschriebenen Verfahren] wie folgt bestimmt:

Ratten werden mit der Testsubstanz oder einem Trägermaterial behandelt; nach 2 Stunden wir ihnen Ionophor A 23187 (0,5 mg/kg) intravenös injiziert. Zwei Minuten nach der Ionophor-Verabreichung wird den Tieren für die Analyse Blut entnommen. Eine bestimmte Einzelmenge jeder Plasmaprobe wird mittels Radioimmunoassay auf Thromboxan $B_2$, und eine weitere auf 6-Keto-$PGF_1\alpha$, die stabilen Metaboliten des Thromboxans $A_2$ bzw. des Prostacyclins ($PGI_2$), untersucht.

Die Verbindungen der Formel I sind sehr wirksame und selektive Thromboxan-Synthetase-Inhibitoren. Bei wirksamen Dosis-Spiegeln und darüber wird weder das vorteilhafte Prostacyclin-Synthetase- noch das Prostaglandin-cyclooxygenase-Enzymsystem wesentlich gehemmt. Ueberraschenderweise wird der Prostacyclin-Spiegel signifikant erhöht.

Bei einer oralen Dosis von 0,04 mg/kg oder weniger senken Verbindungen der Erfindung bei der Ratte die Plasmakonzentration von Thromboxan $B_2$ um über 50 %; gleichzeitig wird ein ungefähr 5-facher oder höherer Anstieg des Plasmaspiegels von Prostacyclin beobachtet.

Aufgrund der genannten vorteilhaften Eigenschaften sind die Verbindungen der Erfindung für Säuger, einschliesslich Menschen, als spezifische therapeutische Mittel sehr wertvoll.

Die Nützlichkeit der Verbindungen der Erfindung bei Thromboembolien ist daraus zu ersehen, dass sie auch die Blutungszeit bei der Ratte verlängern, wenn man sie in einer oralen Dosis von ungefähr 1,0 mg/kg p.o. oder weniger verabreicht.

Die Verbindungen der Erfindung zeigen ferner Lipoxygenase-Hemmung, was auf eine antiinflammatorische Wirksamkeit hindeutet. Die Lipoxygenase-Hemmung lässt sich wie folgt nachweisen:

Peritoneale Neutrophile von Meerschweinchen, hervorgeholt durch eine intraperitoneale Injektion von Natriumcaseinat 17 Stunden vor der Entnahme, werden zusammen mit Indomethacin (1μM) und der Testverbindung 5 Minuten bei 37°C präinkubiert. $^{14}C$-Arachidonsäure (4μM) und Calcium-Ionophor A 23187 (2μM) werden hinzugegeben, und die Proben weitere 5 Minuten bei 37°C inkubiert. Durch Zugabe von 1-n. Salzsäure wird die Reaktion beendet. Die Produkte werden mit Essigsäureäthylester extrahiert und mittels Dünnschichtchromatographie getrennt. Die Platten werden untersucht, die $LTB_4$ und 5-HETE entsprechenden radioaktiven Zonen abgekratzt und in Szintillationsröhrchen für Flüssigkeiten übertragen, worauf ihre Radioaktivität gezählt wird.

Ausser den oben genannten pharmazeutisch verwendbaren Salzen bilden auch alle pharmakologischen Vorstufen der erfindungsgemässen Carbonsäuren, sogenannte "prodrugs", z.B. ihre pharmazeutisch

verwendbaren Ester und Amide, die durch Solvolyse oder unter physiologischen Bedingungen in die genannten Carbonsäuren übergehen können, einen weiteren Gegenstand dieser Erfindung.

Solche Ester sind vorzugsweise z.B. geradkettige oder verzweigte Niederalkylester, die unsubstituiert oder geeignet substituiert sein können, wie etwa Pivaloyloxymethyl-, 2-Diäthylaminoäthyl-, Bornyloxycarbonylmethyl-, α-Carboxyäthyl- oder in geeigneter Weise veresterte α-Carboxyäthylester und dergleichen, welche in an sich bekannter Weise hergestellt werden können.

Die genannten Amide sind vorzugsweise z.B. einfache primäre oder sekundäre Amide und solche, welche von Aminosäuren oder deren Derivaten, z.B. von Alanin oder Phenylalanin, abgeleitet sind.

Bevorzugt sind Verbindungen der Formel I, worin Ar für 3-Pyridyl steht, $R_1$ Wasserstoff, Halogen, Trifluormethyl, Niederalkyl, Niederalkoxy, Niederalkylthio, Hydroxy oder Niederalkanoyloxy bedeutet, p für 1 steht, $R_2$ Wasserstoff oder Niederalkyl ist, $R_3$ Wasserstoff bedeutet und $R_4$ die Gruppe A-B darstellt, in der A die oben angegebene Bedeutung hat und B Carboxy, Niederalkoxycarbonyl, Carbamoyl, Cyan, Hydroxycarbamoyl oder 5-Tetrazolyl bedeutet; und ihre Salze, insbesondere ihre pharmazeutisch verwendbaren Salze.

Von den Verbindungen der Formel I sind weiterhin solche bevorzugt, in denen p für 1 steht, $R_1$ in der 5-Stellung des Indolkerns angeknüpft ist, $R_2$ Wasserstoff oder Niederalkyl bedeutet, $R_3$ Wasserstoff ist und $R_4$ eine Gruppe A-B bedeutet, die in der 3-Stellung des Indolkerns angeknüpft ist.

Von den obengenannten Verbindungen der Formel I sind ferner solche bevorzugt, in denen B für Carboxy, Niederalkoxycarbonyl, Carbamoyl, 5-Tetrazolyl oder Hydroxycarbamoyl steht.

Besonders hervorzuheben sind die obengenannten Verbindungen der Formel I, worin $R_4$ die Gruppe A-B bedeutet, in der A für Alkylen oder Alkenylen mit jeweils 3 bis 10 Kohlenstoffatomen, Niederalkylenphenylen mit 7 bis 10 Kohlenstoffatomen, Niederalkylen-thiophenylen mit 7 bis 10 Kohlenstoffatomen oder Niederalkylen-oxyphenylen mit 7 bis 10 Kohlenstoffatomen steht und B Carboxy oder Niederalkoxycarbonyl bedeutet, $R_1$ Wasserstoff, Niederalkyl, Halogen, Trifluormethyl, Hydroxy, Niederalkylthio oder Niederalkoxy ist, p für 1 steht, Ar 3-Pyridyl bedeutet, $R_2$ Wasserstoff oder Niederalkyl ist und $R_3$ Wasserstoff bedeutet; und ihre Salze, insbesondere ihre pharmazeutisch verwendbaren Salze.

In erster Linie sind Verbindungen der Formel I bevorzugt, worin A für Alkylen mit 3 bis 8 Kohlenstoffatomen steht.

Von besonderem Interesse sind Verbindungen der Formel II

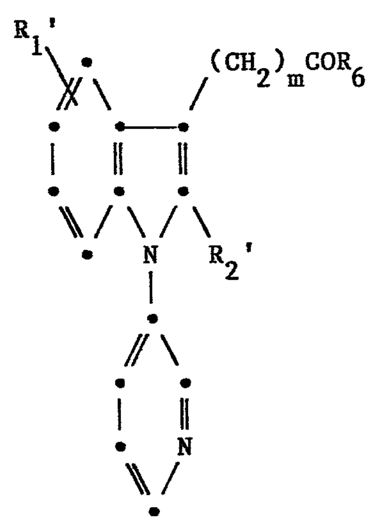

(II),

worin $R_1'$ Wasserstoff, Niederalkyl, Halogen, Trifluormethyl, Hydroxy, Niederalkylthio oder Niederalkoxy bedeutet, $R_2'$ Wasserstoff oder Niederalkyl ist, m für eine ganze Zahl von 1 bis 10 steht und $R_6$ Hydroxy, Niederalkoxy oder Amino bedeutet; und ihre Salze, insbesondere ihre pharmazeutisch verwendbaren Salze.

Weiterhin sind solche Verbindungen der Formel II bevorzugt, worin $R_1'$ Wasserstoff, Methyl, Chlor, Fluor, Trifluormethyl, Hydroxy, Methylthio oder Methoxy bedeutet, $R_2'$ Wasserstoff ist, m für eine ganze Zahl von 3 bis 8 steht und $R_6$ Hydroxy, Aethoxy, Methoxy oder Amino bedeutet; und ihre Salze, insbesondere ihre pharmazeutisch verwendbaren Salze.

Von den letzteren Verbindungen der Formel II sind solche ganz besonders bevorzugt, in denen $R_1'$ Wasserstoff oder Halogen bedeutet, und ihre Salze, insbesondere ihre pharmazeutisch verwendbaren Salze.

In allererster Linie sind Verbindungen der Formel II bevorzugt, worin $R_1'$ Wasserstoff oder Chlor bedeutet, $R_2'$ Wasserstoff ist, m für 4 oder 5 steht und $R_6$ Hydroxy bedeutet; und ihre Salze, insbesondere ihre pharmazeutisch verwendbaren Salze.

Bevorzugt sind ferner Verbindungen der Formel III

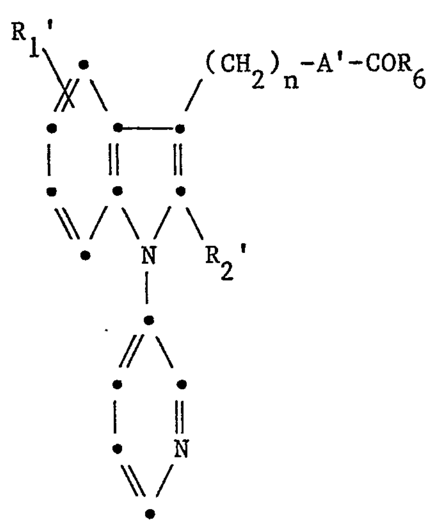

(III),

worin $R_1'$ Wasserstoff, Niederalkyl, Halogen, Trifluormethyl, Hydroxy, Niederalkylthio oder Niederalkoxy bedeutet, $R_2'$ Wasserstoff oder Niederalkyl ist, $R_6$ Hydroxy, Niederalkoxy oder Amino bedeutet, n für eine ganze Zahl von 1 bis 4 steht und A' (Thio oder Oxy)-alkylen mit 1 bis 4 Kohlenstoffatomen, (Thio- oder Oxy)-1,4-phenylen,

1,4-Phenylen, Aethenylen oder Niederalkyl-substituiertes Aethenylen bedeutet; und ihre Salze, insbesondere ihre pharmazeutisch verwendbaren Salze.

Ganz besonders bevorzugt sind Verbindungen der Formel III, worin A' Aethenylen oder Niederalkyl-substituiertes Aethenylen bedeutet und n für 2 oder 3 steht; und ihre Salze insbesondere ihre pharmazeutisch verwendbaren Salze.

Vor allen Dingen bevorzugt sind die in den Beispielen beschriebenen Verbindungen der Formel I und ihre pharmazeutisch verwendbaren Salze.

Die Verbindungen der vorliegenden Erfindung werden nach an sich bekannten Methoden, vorzugsweise dadurch hergestellt, dass man z.B.

1) eine Verbindung der Formel IV

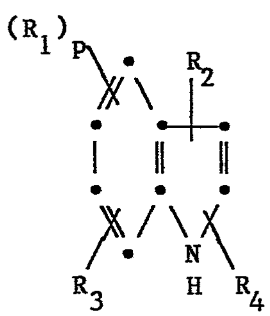

(IV),

worin $R_1$, $R_2$, $R_3$, $R_4$ und p die unter Formel I angegebene Bedeutung haben, mit einer Verbindung der Formel

Ar-X ,

worin Ar die unter Formel I angegebene Bedeutung hat und X für reaktives verestertes Hydroxy steht, umsetzt, oder

2) eine Verbindung der Formel V

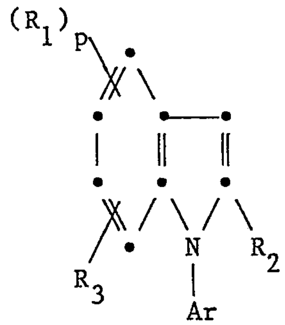

(V)

oder ein 3-Halogen-Derivat davon, worin Ar, $R_1$, p und $R_2$ die unter Formel I angegebene Bedeutung haben und $R_3$ Wasserstoff ist, mit einem reaktiven funktionellen Derivat einer Verbindung der Formel VI

$$HO - A - B \qquad (VI),$$

worin A und B die unter Formel I angegebene Bedeutung haben, umsetzt, oder

3) eine Verbindung der Formel VII

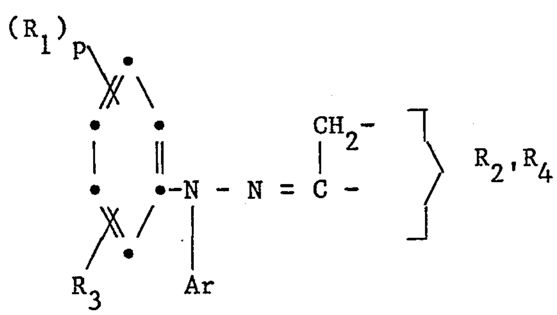

(VII),

die einen Rest $R_2$ und einen Rest $R_4$ enthält und worin $R_1$, $R_2$, $R_3$, $R_4$, p und Ar die unter Formel I angegebene Bedeutung haben, ringschliesst, oder

4) einer Verbindung der Formel VIII

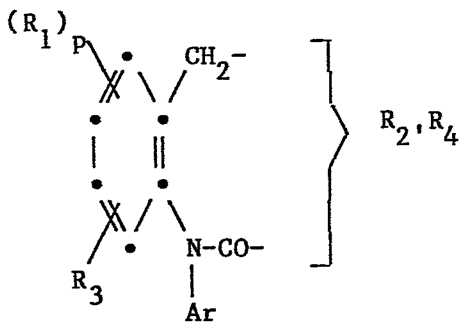

(VIII),

die eine Gruppe $R_2$ und eine Gruppe $R_4$ enthält und worin $R_1$, $R_2$, $R_3$, $R_4$, p und Ar die unter Formel I angegebene Bedeutung haben, cyclisiert oder

5) eine Verbindung der Formel IX

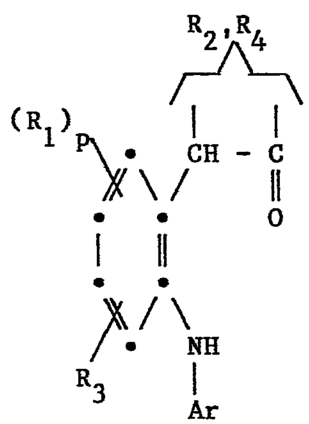

(IX),

die einen Rest $R_2$ und einen Rest $R_4$ enthält und worin $R_1$, $R_2$, $R_3$, $R_4$, p und Ar die unter Formel I angegebene Bedeutung haben, cyclisiert, oder

6) eine Verbindung der Formel Ia

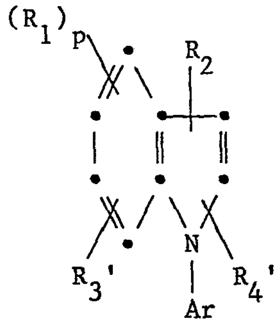

(Ia),

worin Ar, $R_1$, p und $R_2$ die unter Formel I angegebene Bedeutung haben, einer der beiden Reste $R_3'$ und $R_4'$ Wasserstoff und der jeweils andere die Gruppe A-C bedeutet, in der C eine von B verschiedene und in B überführbare Gruppe bedeutet, in eine Verbindung der Formel I umwandelt, gegebenenfalls unter Ausdehnung der Kette A innerhalb ihrer Definition, oder

7) eine Verbindung der Formel X

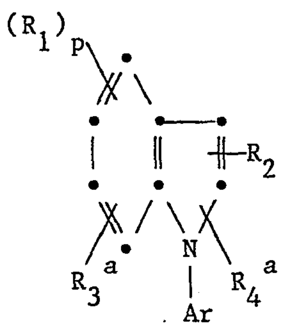

(X),

worin Ar, $R_1$, p und $R_2$ die unter Formel I angegebene Bedeutung haben, einer der beiden Reste $R_3^a$ und $R_4^a$ Wasserstoff bedeutet und der jeweils andere für Formyl oder Niederalkanoyl steht, mit einer Verbindung der Formel XI

$$R_5 - A'' - B \qquad (XI),$$

worin A" die unter Formel I für A definierte Bedeutung hat, jedoch die Kettenlänge um ein Kohlenstoffatom verkürzt ist, und $R_5$ einen Dialkylphosphono- oder Triarylphosphonium-Rest bedeutet; und, falls gewünscht, im erhaltenen Produkt der Formel I die Doppelbindung, welche direkt am Indolring sitzt, reduziert, oder

8) eine Verbindung der Formel XII

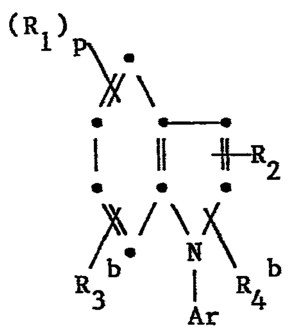

(XII),

worin Ar, $R_1$, p und $R_2$ die unter Formel I angegebene Bedeutung haben, einer der Reste $R_3^b$ und $R_4^b$ Wasserstoff bedeutet und der jeweils andere für reaktives verestertes Hydroxy steht, mit einer Verbindung der Formel XIII

$$CH_2=CH-A'''-B \qquad (XIII),$$

worin A"' die unter Formel I für A definierte Bedeutung hat, worin jedoch die Kettenlänge um 2 Kohlenstoffatome verkürzt ist, oder worin A"' eine direkte Bindung bedeutet, umsetzt, und, wenn gewünscht, im erhaltenen Produkt der Formel I die Doppelbindung, die direkt am Indolring sitzt, reduziert; oder

9) eine Verbindung der Formel XVII

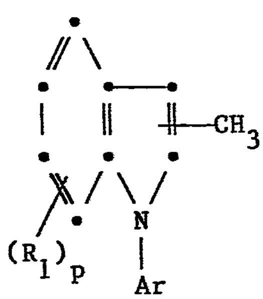

(XVII),

worin Ar, $R_1$ und p die unter Formel I angegebene Bedeutung haben, mit einem reaktiven funktionellen Derivat einer Verbindung VI'

HO - A" - B (VI'),

worin A" die unter Formel I für A angegebene Bedeutung hat, jedoch die Kettenlänge um ein Kohlenstoffatom verkürzt ist, und B die unter Formel I definierte Bedeutung hat, umsetzt; wobei störende reaktive Gruppen gegebenenfalls vorübergehend geschützt sind; und/oder, wenn erwünscht, eine erhaltene Verbindung der Formel I in eine andere Verbindung der Erfindung umwandelt, und/oder, wenn erwünscht, eine erhaltene freie Verbindung der Formel I in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

Zur Durchführung des Verfahrens 1) wird eine Verbindung der Formel IV mit einer Verbindung Ar-X, worin X vorzugsweise Halogen und insbesondere Brom bedeutet, umgesetzt, vorteilhaft in Gegenwart eines Katalysators für die Ullmann-Reaktion [s. Organic Reactions 14, 19 (1965)], z.B. Kupfer, Kupferbronze, Kupferoxid, Triphenylphosphin-Nickel, vorzugsweise in einem polaren Lösungsmittel wie z.B. Pyridin oder Dimethylformamid, gegebenenfalls in Gegenwart einer nicht-wässerigen Base wie z.B. Kaliumcarbonat, bei einer Temperatur zwischen ungefähr 75° und 150°C, z.B. bei 100°C, oder wie in J. Chem. Soc. (C) 1970, 85-91 beschrieben.

Die Indol-Ausgangsverbindungen der Formel IV sind bekannt oder, wenn neu, werden sie in an sich bekannter Weise, z.B. gemäss der Fischer-Indolsynthese oder wie in den Beispielen beschrieben, hergestellt.

Zur Durchführung des Verfahrens 2) werden die Verbindungen der Formel V oder ihre 3-Halogen-Derivate zunächst in reaktive metallorganische Derivate überführt, z.B. in Alkalimetall- oder Halogenmagnesium-(Grignard)-Derivate mittels eines zur Metallierung geeigneten Agens, z.B. einem Grignard-Reagens, einer Alkalimetallbase oder einer quaternären Ammoniumbase. Vorzugsweise werden die Verbindungen der Formel V in situ in die reaktiven metallorganischen Zwischenprodukte unter Verwendung eines reaktiven metallierenden Agens umgewandelt, vorzugsweise mit einem molaren Aequivalent von z.B. einer starken Alkalimetallbase, wie z.B. Butyllithium, Lithiumdiisopropylamid, Natriumhydrid, Kalium-tert.-butoxid, einem Grignard-Reagens, z.B. einem Niederalkylmagnesiumhalogenid wie Methylmagnesiumbromid, in einem inerten Lösungsmittel, wie z.B. Dimethylformamid, Diäthyläther oder Tetrahydrofuran, in einem Temperaturbereich zwischen -50° bis +75°C, vorzugsweise zwischen -25° und +50°C. Die Umsetzung der erhaltenen reaktiven metallorganischen Verbindung der Formel V mit einem reaktiven veresterten Derivat einer Verbindung der Formel VI wird in einem Temperaturbereich von ungefähr -25° bis +50°C, vorzugsweise in einem Bereich von 0° bis 30°C durchgeführt. In den Fällen, wo B für Carboxy, Carbamoyl, Hydroxycarbamoyl oder Mono-niederalkylcarbamoyl steht, sind eines oder mehrere weitere molare Aequivalente des Metallierungsmittels erforderlich.

Die Zwischenprodukte der Formel V sind bekannt (z.B. J. Chem. Soc. (C) 1970, 85) oder werden analog zu den bekannten hergestellt, z.B. aus den entsprechenden gegebenenfalls substituierten Indolen, wie in den Beispielen beschrieben.

Die Ausgangsverbindungen der Formel VI sind bekannt oder, wenn neu, werden sie nach herkömmlichen Methoden hergestellt, z.B. gemäss den im US-Patent 4.256.757 und in der Britischen Patentanmeldung 2.016.452 A angegebenen Verfahrensweisen.

Der Ringschluss des Ausgangsstoffes der Formel VII gemäss dem Verfahren 3) wird gemäss der wohlbekannten Fischer-Indolsynthese durchgeführt, die z.B. in "Heterocyclic Compounds, Indoles Part I" (Herausgeber: W.J. Houlihan) S. 232-317, beschrieben ist, und zwar thermisch oder vorzugsweise in Gegenwart eines sauren Kondensationsmittels. Im letzeren Fall arbeitet man vorzugsweise in Gegenwart von Halogenwasserstoffen, z.B. äthanolischem Chlorwasserstoff, oder Polyphosphorsäure, gegebenenfalls in einem inerten Lösungsmittel, vorzugsweise bei Temperaturen zwischen ungefähr 50° und 100°C.

Die Hydrazon-Zwischenprodukte der Formel VII werden - in isolierter Form oder vorzugsweise in situ - durch Kondensation eines Ketons der Formel $R_2CH_2COR_4$ oder $R_4CH_2COR_2$, worin $R_2$ und $R_4$ die unter Formel I angegebene Bedeutung haben, mit einem substituierten Hydrazin der Formel XIV

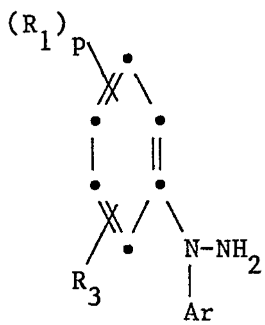

(XIV),

worin $R_1$, p, Ar und $R_3$ die unter Formel I angegebene Bedeutung haben, vorzugsweise in Gegenwart eines sauren Katalysators, hergestellt.

Die Hydrazin-Ausgangsstoffe der Formel XIV sind bekannt oder werden vorzugsweise z.B. durch Nitrosierung von entsprechend substituierten Anilinen der Formel XV

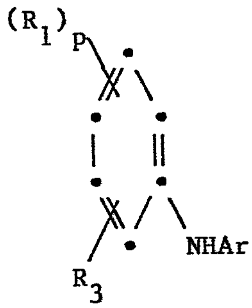

(XV),

worin die Symbole $R_1$, p, Ar und $R_3$ die vorher angegebene Bedeutung haben, und nachfolgende Reduktion der N-Nitroso-Derivate, z.B. mit Zink in Essigsäure oder nach anderen an sich bekannten Methoden, hergestellt.

Die Cyclisierung gemäss dem Verfahren 4) wird unter den Bedingungen der Indolsynthese nach Madelung, wie in "Heterocyclic Compounds, Indoles Part I" (Herausgeber W.J. Houlihan) S. 385-396, beschrieben, vorgenommen. Die intramolekulare Cyclisierung wird vorzugsweise in Gegenwart einer starken Base, z.B. Natrium-äthoxid, Natriumamid oder Kalium-tert.-butoxid, vorteilhaft bei erhöhter Temperatur, z.B. bei ungefähr 300°C, oder in einem inerten hochsiedenden Lösungsmittel, z.B. Tetrahydronaphthalin, durchgeführt.

Die Ausgangsstoffe der Formel VIII werden durch Acylierung von substituierten Anilinen der Formel XVI und XVIa

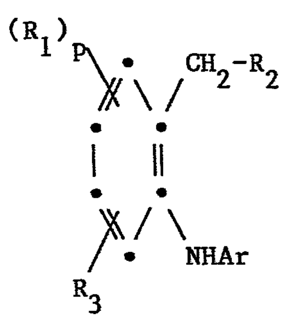

(XVI)

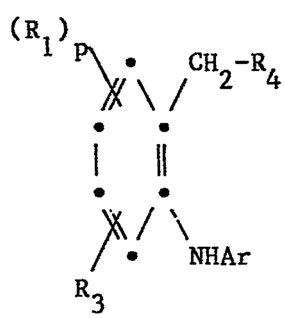

(XVIa)

worin $R_1$, $R_2$, $R_3$, $R_4$, p und Ar die oben angegebene Bedeutung haben, mit einer Verbindung der Formel $R_4COOH$ bzw. $R_2COOH$ oder einem reaktionsfähigen funktionellen Derivat davon, erhalten.

Die Cyclisierung gemäss Verfahren 5) kann in Gegenwart einer starken Base, z.B. Kalium-tert.-butoxid oder Natriumhydrid, in einem polaren Lösungsmittel, z.B. Dimethylformamid oder Dimethoxyäthan, durchgeführt werden.

Die Umwandlung einer Verbindung der Formel Ia, worin sich C von B unterscheidet, gemäss dem Verfahren 6) in eine Verbindung der Formel I, und die fakultative Umwandlung eines erhaltenen Produkts der Formel I in eine andere Verbindung dieser Erfindung, werden gemäss an sich bekannten chemischen Methoden und/oder wie hier beschrieben vorgenommen.

Umwandelbare Gruppen C sind vorzugsweise Trialkoxymethyl, Hydroxymethyl, verestertes Hydroxymethyl, veräthertes Hydroxymethyl, Halogenmethyl, 2-Oxazolinyl mit Ausnahme von 4,5-Dihydro-2-oxazolyl, Dihydro-2-oxazolinyl, Niederalkanoyloxymethyl, Acetyl, Methyl, Carboxycarbonyl, Formyl, Trihalogenacetyl, Di-niederalkoxymethyl, Alkylendioxymethyl, Vinyl, Alkinyl, verestertes Carboxy und amidiertes Carboxy.

Die Zwischenprodukte der Formel Ia werden z.B. in strenger Analogie zu den Verfahren 1) bis 8), die auf die Herstellung von Verbindungen der Formel I gerichtet sind, oder wie hier beschrieben, unter Verwendung von an sich bekannten chemischen Methoden, hergestellt.

Die Zwischenprodukte der Formel Ia sind ebenfalls wirksam als Thromboxan-Synthetase-Hemmer. Bevorzugt sind solche Zwischenprodukte der Formel Ia, worin A Alkylen mit 3 bis 8, insbesondere mit 4 oder 5, Kohlenstoffatomen bedeutet und C für Hydroxymethyl oder Formyl steht.

Die Kondensationsreaktion gemäss Verfahren 7) wird unter Bedingungen wie sie bei der Wittig-Reaktion üblich sind, z.B. wie in J. Am. Chem. Soc. 83, 1733 (1961) beschrieben, unter Bildung eines Ylids, z.B. in Gegenwart einer starken Base wie z.B. Natriumhydrid,

in einem Lösungsmittel wie z.B. Methylenchlorid oder Toluol, bei einer Temperatur zwischen -20° und +100°C, vorzugsweise zwischen -10° und +50°C, durchgeführt.

Die Reaktion gemäss Verfahren 8) wird z.B. so durchgeführt wie in Accounts of Chemical Research 12, 146 (1979) beschrieben, in Gegenwart z.B. eines Triarylphosphins, z.B. Tri-o-tolylphosphin, und einem Palladiumsalz, z.B. Palladiumacetat, und einer Base, z.B. Triäthylamin, in einem polaren Lösungsmittel, z.B. der genannten Base oder Acetonitril, vorzugsweise bei einer Temperatur zwischen 25° und 100°C, wobei man zunächst eine Verbindung der Erfindung erhält, in der die Gruppe $R_3$ oder $R_4$ eine direkt am Indolring sitzende Doppelbindung enthält.

In den Ausgangsverbindungen der Formel XII ist reaktives verestertes Hydroxy vorzugsweise Halogen, kann aber auch eine der unten angegebenen Bedeutungen haben. Die Verbindungen der Formel XII, z.B. solche, in denen einer der Reste $R_3^b$ oder $R_4^b$ Brom bedeutet, werden nach an sich bekannten Methoden, z.B. wie in Can. J. Chemistry 41, 2399 (1963) beschrieben, hergestellt.

Die Reaktion gemäss Verfahren 9) wird unter den gleichen Bedingungen wie für Verfahren 2) beschrieben unter Verwendung eines geeigneten Metallierungsmittels wie z.B. Lithiumdiisopropylamid durchgeführt.

Die einzelnen Definitionen in den vorher beschriebenen Verfahren haben folgende Bedeutungen:

Reaktives verestertes Hydroxy, z.B. in einem reaktionsfähigen funktionellen Derivat eines Alkohols der Formel VI, ist z.B. Hydroxy, welches mit einer starken anorganischen oder organischen Säure, vor allem mit einer Halogenwasserstoffsäure, z.B. Chlorwasserstoff-, Bromwasserstoff- oder Jodwasserstoffsäure, einer

aliphatischen oder aromatischen Sulfonsäure, z.B. Methansulfon- oder p-Toluolsulfonsäure, verestert ist. Diese Verbindungen werden in an sich bekannter Weise hergestellt.

Trialkoxymethyl bedeutet vorzugsweise Tri(niederalkoxy)-methyl, insbesondere Triäthoxy- oder Trimethoxy-methyl.

Veräthertes Hydroxymethyl bedeutet vorzugsweise tertiäres Niederalkoxymethyl, niederes Alkoxy-alkoxymethyl, z.B. Methoxymethoxymethyl, 2-Oxa- oder 2-Thiacycloalkoxymethyl, insbesondere 2-Tetrahydropyranyloxymethyl.

Verestertes Hydroxymethyl bedeutet vorzugsweise Niederalkanoyloxymethyl, vorteilhaft Acetoxymethyl, Niederalkylsulfonyloxymethyl, z.B. Methylsulfonyloxymethyl, oder Arylsulfonyloxymethyl, z.B. p-Tolylsulfonyloxymethyl.

Halogenmethyl steht insbesondere für Chlormethyl, kann aber auch Brommethyl oder Jodmethyl sein.

Ein Alkalimetall bedeutet vorzugsweise Lithium, kann aber auch Kalium oder Natrium sein.

Die Umwandlung von Verbindungen der Formel Ia in Verbindungen der Formel I sowie die Umwandlung von Verbindungen der Formel I in eine andere Verbindung der Erfindung werden nach an sich bekannten chemischen Methoden durchgeführt.

Verbindungen der Formel I und Zwischenprodukte, in denen $R_1$ Aryl-niederalkoxy, z.B. Benzyloxy, oder Niederalkoxy, z.B. Methoxy, bedeutet, könnnen durch Hydrierung bzw. Hydrolyse in an sich bekannter Weise in entsprechende Verbindungen, worin $R_1$ Hydroxy ist, überführt werden.

Die Verbindungen der Formel I, worin B Hydroxycarbamoyl bedeutet (Hydroxamsäuren), werden vorzugsweise dadurch erhalten, dass man eine Verbindung der Formel I, worin B Carboxy oder ein reaktionsfähiges funktionelles Derivat davon, Niederalkoxycarbonyl oder Carbamoyl bedeutet, mit Hydroxylamin oder einem Säureadditionssalz davon in Gegenwart einer Base, z.B. Natriumhydroxid, umsetzt.

Diese Reaktion wird in an sich bekannter Weise durchgeführt, z.B. wie von Barton et al., Comprehensive Organic Chemistry, Vol. 2, S. 1037-1038 (1979) beschrieben, vorzugsweise unter basischen Bedingungen, vorteilhaft mit Hydroxylamin-Hydrochlorid, in einem inerten polaren Lösungsmittel, z.B. einem Niederalkanol wie Aethanol, vorzugsweise in einem Temperaturbereich von 0° bis 50°C und vorteilhaft bei Raumtemperatur.

Verbindungen der Formel I, worin B 5-Tetrazolyl bedeutet, werden vorzugsweise dadurch hergestellt, dass man eine Verbindung der Formel I, worin B vorzugsweise Cyan ist, mit Stickstoffwasserstoffsäure oder einer Verbindung, die Stickstoffwasserstoffsäure in situ bildet, z.B. einem Metall- oder Ammoniumsalz dieser Säure, vorzugsweise einem Alkalimetallazid wie z.B. Natriumazid oder Ammoniumazid, umsetzt.

Diese Kondensation wird in an sich bekannter Weise durchgeführt, z.B. wie von Barton et al., Comprehensive Organic Chemistry, Vol. 4, S. 407 - 409 (1979) beschrieben, vorzugsweise in einem Lösungsmittel, z.B. Dimethylformamid, und bei erhöhter Temperatur, etwa zwischen 50° und 200°C, vorteilhaft zwischen 75° und 150°C, und in Gegenwart einer Säure, z.B. Salzsäure oder Ammoniumchlorid.

Die genannten Tetrazolyl-Verbindungen können weiterhin aus einer Verbindung der Formel I, worin C Cyan oder Carbamoyl ist, hergestellt werden, indem der Rest C zunächst in Halogen- oder Niederalkoxyiminocarbonyl überführt und dann z.B. mit einem Alkalimetall- oder Ammoniumazid umgesetzt wird.

Verbindungen der Formel I, worin B unsubstituiertes oder durch Niederalkyl substituiertes 4,5-Dihydro-2-oxazolyl bedeutet, werden vorzugsweise durch die Umsetzung einer Verbindung der Formel I, worin B für Carboxy oder ein reaktives funktionelles Derivat davon, Niederalkoxycarbonyl oder Carbamoyl steht, mit 2-Hydroxyäthylamin, das gegebenenfalls durch Niederalkyl mono- oder di-(vicinal oder geminal)-C-substituiert sein kann, oder mit Aziridin, das gegebenenfalls durch Niederalkyl mono- oder di-(vicinal oder geminal)-C-substituiert sein kann, z.B. 2-Aminoäthanol, 2-Methyl-2-aminopropanol oder 2,2-Dimethylaziridin, hergestellt.

Die Kondensation wird nach an sich bekannten Methoden durchgeführt, z.B. wie in J. Org. Chem. 39, 2787 (1974) beschrieben, vorzugsweise in einem inerten Lösungsmittel, z.B. Toluol, in einem Temperaturbereich von 25° bis 100°C.

Die erwähnte Kondensationsreaktion läuft entweder spontan ab, oder, z.B. in dem Fall, wo B für Carboxy steht, in Gegenwart von Kondensationsmitteln, z.B. disubstituierten Carbodiimiden wie etwa Dicyclohexylcarbodiimid.

Zwischenprodukte der Formel Ia, in welchen C Halogenmethyl bedeutet, können vorzugsweise mit einem Metallcyanid, z.B. Kaliumcyanid, in an sich bekannter Weise umgesetzt werden. Man erhält dabei Verbindungen der Formel I, in welchen die Kette um ein Kohlenstoffatom verlängert ist und B für Cyan steht. Diese können ihrerseits nach an sich bekannten Methoden in Verbindungen der Formel I, worin B Carboxy, Niederalkoxycarbonyl oder Carbamoyl bedeutet, überführt werden.

So können Verbindungen der Formel I, in welchen B Cyan bedeutet (Nitrile), in Verbindungen der Formel I, worin B für Carboxy steht, durch Hydrolyse mit anorganischen Säuren, z.B. mit Halogenwasserstoffsäuren, wie Chlorwasserstoffsäure, oder Schwefelsäure in wässeriger Lösung, oder vorzugsweise durch Hydrolyse mit wässerigen Alkalimetallhydroxiden, z.B. Kaliumhydroxid, bei Rückflusstemperatur umgewandelt werden.

Die Ueberführung der genannten Nitrile in Verbindungen der Formel I, worin B Niederalkoxycarbonyl bedeutet, wird vorzugsweise zunächst durch Behandlung mit einem Niederalkanol, z.B. wasserfreiem Aethanol, in Gegenwart einer starken Säure, z.B. Chlorwasserstoffsäure, vorzugsweise unter Rückfluss, und nachfolgende vorsichtige Hydrolyse mit Wasser vorgenommen.

Die Ueberführung der genannten Nitrile in Verbindungen der Formel I, worin B für Carbamoyl steht, wird vorzugsweise durch Behandlung mit einem Alkalimetallhydroxid, z.B. verdünnter Natronlauge, und Wasserstoffperoxid, vorzugsweise bei Raumtemperatur, durchgeführt.

Zwischenprodukte der Formel Ia, in welchen C Halogenmethyl, z.B. Chlormethyl, bedeutet, können in Verbindungen der Formel I, worin B für Carboxy steht und die Kette um 2 Kohlenstoffatome verlängert ist, umgewandelt werden, indem sie zunächst z.B. mit einem Diniederalkyl-malonat, z.B. Diäthylmalonat, in Gegenwart einer Base, z.B. Kaliumcarbonat oder Natriumäthoxid, in einem Lösungsmittel wie Dimethylformamid, vorzugsweise bei einer Temperatur zwischen 50° und 100°C behandelt werden. Die entstandenen substituierten Di-niederalkylmalonate werden dann vorzugsweise mit einer wässerigen Base, z.B. verdünnter Natronlauge, zur entsprechenden Malonsäure hydrolysiert, welche unter Standardbedingungen, z.B. durch Erhitzen in Xylol, zu einer Verbindung der Formel I, worin B Carboxy bedeutet, decarboxyliert wird. Ersetzt man den Malonsäure-di-niederalkylester durch einen Cyanessigsäure-niederalkylester, so erhält man die entsprechenden Verbindungen der Formel I, in welchen B Cyan bedeutet.

Verbindungen der Erfindung, in denen A ein geradkettiger oder verzweigter Alkenylenrest mit einer terminalen Doppelbindung ist, können auch aus den Zwischenprodukten der Formel Ia, worin C Halogenmethyl bedeutet, hergestellt werden. So können z.B. diese Zwischenprodukte zuerst mit einem Niederalkylester einer α-(Aryl- oder Alkyl)-thioessigsäure, z.B. α-(Phenylthio)-essigsäureäthyl-

ester, in Gegenwart einer starken Base, z.B. Natriumhydrid, behandelt werden. Die nachfolgende Oxidation des erhaltenen α-Arylthio- oder α-Alkylthio-substituierten Esters zum entsprechenden α-Arylsulfinyl- oder α-Alkylsulfinyl-ester z.B. mit Natriumperjodat, gefolgt von einer durch Hitze ausgelösten Eliminierung, z.B. durch Erhitzen in Xylol, ergibt eine Verbindung der allgemeinen Formel I (einen α,ß-ungesättigten Ester), in der A Alkenylen bedeutet und B z.B. Niederalkoxycarbonyl ist. Die Kette wird gleichzeitig um zwei Kohlenstoffatome verlängert. Die gleiche Umwandlung kann auch unter Verwendung z.B. von α-(Phenylseleno)-essigsäure-äthylester, wie in J. Am. Chem. Soc. 95, 6137 (1973) beschrieben, durchgeführt werden. Auch können die Verbindungen der Formel Ia, in denen C Halogenmethyl bedeutet, zuerst in die entsprechenden Carboxyaldehyde umgewandelt werden, z.B. mit Dimethylsulfoxid in Gegenwart von Triäthylamin und Silbertetrafluoroborat, oder mit Chromtrioxid und Pyridin in Methylenchlorid. Die nachfolgende Wittig-Kondensation z.B. mit Trimethylphosphonoacetat oder (Triphenylphosphoranyliden)-essigsäure-äthylester ergibt ebenfalls die obengenannten α,ß-ungesättigten Ester.

Verbindungen der Formel I, worin B Niederalkoxycarbonyl ist, können mit Ammoniak, Mono- oder Di-niederalkylaminen, z.B. Methylamin oder Dimethylamin, in einem inerten Lösungsmittel, z.B. einem Niederalkanol wie Butanol, gegebenenfalls bei erhöhten Temperaturen, zu Verbindungen der Formel I, in welchen B unsubstituiertes, mono- oder di-niederalkyl-substituiertes Carbamoyl bedeutet, amidiert werden.

Verbindungen der Formel I, worin A einen geradkettigen oder verzweigten Alkenylenrest mit einer terminalen Doppelbindung bedeutet, z.B. α,ß-ungesättigte Ester, können auch aus den entsprechenden α,ß-gesättigten Verbindungen durch Behandlung z.B. mit Phenylselenylchlorid in Gegenwart einer starken Base gemäss der in J. Am. Chem. Soc. 95, 6137 (1973) beschriebenen Methode erhalten werden.

Die Umwandlung von Verbindungen der Formel I, worin B Niederalkoxycarbonyl, Cyan, unsubstituiertes, mono- oder di-niederalkyl-substituiertes Carbamoyl oder unsubstituiertes oder durch Niederalkyl substituiertes 4,5-Dihydro-2-oxazolyl bedeutet, in Verbindungen der Formel I, in denen B Carboxy ist, wird vorteilhaft durch Hydrolyse mit anorganischen Säuren, z.B. mit Halogenwasserstoffsäuren oder Schwefelsäure, oder mit wässerigen Alkalien, vorzugsweise mit Alkalimetallhydroxiden, z.B. Lithium- oder Natriumhydroxid, durchgeführt.

Verbindungen der Formel I, in welchen B Carboxy oder Niederalkoxycarbonyl bedeutet, können mit einfachen oder komplexen Leichtmetallhydriden, z.B. mit Lithiumaluminiumhydrid, Alan oder Diboran, zu Verbindungen der Formel Ia, in denen C für Hydroxymethyl steht, reduziert werden. Diese Alkohole können auch durch geeignete Solvolyse von Zwischenprodukten der Formel Ia, worin C Halogenmethyl bedeutet, durch Behandlung z.B. mit einem Alkalimetallhydroxid, z.B. Lithium- oder Natriumhydroxid, erhalten werden.

Die vorher genannten Alkohole können ihrerseits mit konventionellen Oxidationsmitteln, vorzugsweise mit Pyridin-dichromat in Dimethylformamid bei Raumtemperatur, in Verbindungen der Formel I, worin B Carboxy bedeutet, umgewandelt werden.

Freie Carbonsäuren können mit Niederalkanolen, z.B. Aethanol in Gegenwart einer starken Säure, z.B. Schwefelsäure, vorzugsweise bei erhöhter Temperatur, oder mit Diazo-niederalkanen, z.B. Diazomethan in einem Lösungsmittel, z.B. Diäthyläther, vorzugsweise bei Raumtemperatur, zu den entsprechenden Estern, nämlich zu solchen Verbindungen der Formel I, worin B Niederalkoxycarbonyl bedeutet, umgesetzt werden.

Weiter können die freien Carbonsäuren durch Behandlung eines ihrer reaktionsfähigen Zwischenprodukte, z.B. eines Acylhalogenids, etwa eines Säurechlorids oder gemischten Anhydrids, z.B. eines solchen, das von einem Halogenkohlensäure-niederalkylester, z.B. Chlorameisensäure-äthylester abgeleitet ist, mit Ammoniak, Mono- oder Di-niederalkylaminen, in einem inerten Lösungsmittel, z.B. Methylenchlorid, vorzugsweise in Gegenwart eines basischen Katalysators, z.B. Pyridin, in Verbindungen der Formel I, in denen B für unsubstituiertes, mono- oder di-niederalkyl-substituiertes Carbamoyl steht, umgewandelt werden.

Verbindungen der Formel I, worin B Mono-niederalkyl-carbamoyl bedeutet, können in Verbindungen der Formel I, in denen B für Di-niederalkyl-carbamoyl steht, durch Behandlung mit einer starken Base, z.B. Natriumhydrid, gefolgt von einem Alkylierungsmittel, z.B. einem Niederalkylhalogenid, in einem inerten Lösungsmittel, z.B. Dimethylformamid, überführt werden.

Weiter können Verbindungen der Formel I, in denen A einen geradkettigen oder verzweigten Alkinylen- oder Alkenylenrest bedeutet, durch katalytische Hydrierung, vorzugsweise unter neutralen Bedingungen, z.B. mit einem Palladium-Katalysator bei atmosphärischem Druck, in einem inerten Lösungsmittel, z.B. Aethanol, in Verbindungen der Formel I, worin A für geradkettiges oder verzweigtes Alkylen steht, umgewandelt werden.

Carboxaldehyde, nämlich Verbindungen der Formel Ia, in denen C Formyl bedeutet, können durch Oxidation von Verbindungen der Formel Ia, worin C Hydroxymethyl bzw. Halogenmethyl bedeutet, z.B. mit Dimethylsulfoxid und einem Katalysator, z.B. einem Gemisch aus Triäthylamin und Silbertetrafluoroborat, oder mit Chromtrioxid und Pyridin oder mit an sich bekannten anderen geeigneten Oxidationsmitteln hergestellt werden. Die genannten Carboxaldehyde können in die entsprechenden Acetale, d.h. Verbindungen der Formel Ia, worin C Di-(niederalkoxy)-methyl oder Alkylendioxymethyl bedeutet, z.B. das Dimethylacetal, durch säurekatalysierte Kondensation mit einem Alkohol, z.B. Methanol, umgewandelt werden.

Verbindungen der Formel I, worin B Carboxy bedeutet, können durch die wohlbekannte Arndt-Eistert-Synthese zu Verbindungen der Formel I, worin B Carboxy ist und die Kette A ein Kohlenstoffatom mehr enthält, umgewandelt werden. Insbesondere kann man ein reaktionsfähiges funktionelles Derivat der als Ausgangsstoff verwendeten Carbonsäure, z.B. ein Säurechlorid, mit Diazomethan z.B. in Diäthyläther behandeln, wobei man eine Verbindung der Formel Ia, worin C Diazoacetyl bedeutet, erhält. Nach Behandlung z.B. mit Silberoxid erhält man die genannte Carbonsäure der Formel I, worin die Kette A um ein Kohlenstoffatom verlängert ist.

Eine besondere Ausführungsform des Verfahrens 6) betrifft die Herstellung von Verbindungen der Formel I, worin B Carboxy bedeutet. Sie besteht darin, dass man in einer Verbindung der Formel Ia, worin C einen in die Carboxygruppe überführbaren Rest bedeutet, die Gruppe C, gegebenenfalls unter Verlängerung der Kette A im Rahmen ihrer Definition, in Carboxy überführt.

In eine Carboxygruppe überführbare Reste sind z.B. verestertes Carboxy, anhydriertes Carboxy einschliesslich entsprechender asymmetrischer und innerer Anhydride, amidiertes Carboxy, Cyan, Amidin-Strukturen, einschliesslich cyclischer Amidine, wie z.B. 5-Tetrazolyl, Iminoäther, einschliesslich cyclischer Iminoäther, wie z.B. unsubstituierter oder durch Niederalkyl substituierter 2-Oxazolinyl-Reste mit Ausnahme von 4,5-Dihydro-2-oxazolyl, oder unsubstituiertes oder durch Niederalkyl substituiertes oder Dihydro-2-oxazolinyl, ferner Methyl, Hydroxymethyl, veräthertes Hydroxymethyl, Niederalkanoyloxymethyl, Trialkoxymethyl, Acetyl, Trihalogenacetyl, Halogenmethyl, Carboxycarbonyl (-COCOOH), Formyl, Di-niederalkoxymethyl, Alkylendioxymethyl, Vinyl, Aethinyl, oder Diazoacetyl. Gleichzeitig mit der Umwandlung von C in die Carboxygruppe kann die Kette A im Rahmen ihrer Definition verlängert werden.

Verestertes Carboxy ist vorzugsweise Carboxy in Form seiner Niederalkylester, z.B. des Methyl-, Aethyl-, n- oder Iso-(Propyl oder Butyl)-esters. Ferner in Form von substituierten Niederalkylestern, z.B. dem ω-Amino-, ω-Monomethylamino- oder ω-Dimethylamino-, α-Carboxy- oder α-Aethoxycarbonyl-(äthyl, propyl oder butyl)-ester. Weitere Ester sind Aryl-niederalkylester, z.B. Benzyl-, (Methyl, Methoxy, Chlor)-substituierte Benzyl- und Pyridylmethyl-Ester; Niederalkanoyloxy-niederalkylester, z.B. Pivaloyloxymethylester; unsubstituierte und durch Methyl, Methoxy oder Chlor substituierte 3-Phthalidylester, die von den entsprechenden 3-Hydroxy-phthaliden abgeleitet sind, (Hydroxy, Niederalkanoyloxy, Niederalkoxy)-substituierte Niederalkoxy-methylester, z.B. ß-(Hydroxy, Acetyloxy, Methoxy)-äthoxymethylester; Bicycloalkyloxycarbonyl-niederalkylester, z.B. solche, die von bicyclischen Monoterpenoid-Alkoholen, z.B. unsubstituierten oder durch Niederalkyl substituierten Bicyclo[2,2,1]heptyloxycarbonyl-niederalkylestern, vorteilhaft Bornyloxycarbonylmethylestern; oder Halogen-substituierten Niederalkylestern, z.B. Trichloräthyl- oder Jodäthylestern, abgeleitet sind.

Amidiertes Carboxy ist vorzugsweise Carboxy in Form seiner unsubstituierten Amide, N-Mono- oder N,N-Di-niederalkylamide, z.B. Mono-oder Di-methylamide; in Form von tertiären Amiden, die z.B. von Pyrrolidin, Piperidin oder Morpholin abgeleitet sein können; ferner α-Niederalkoxycarbonyl- oder α-Carboxy-substituierte Niederalkylamide, z.B. Mono-N-äthoxycarbonylmethyl)-amide und Mono-N-(carboxymethyl)-amide; α-Niederalkoxycarbonyl- oder α-Carboxy-substituierte Aryl-niederalkylamide, z.B. Aethoxycarbonyl- oder Carboxy-substituierte Phenyläthylamide; Amino-niederalkylamide, z.B. ß-Aminoäthylamide und ß-(Benzyloxycarbonyl-amino)-äthylamide.

Die Ueberführung in die Carboxygruppe wird nach an sich bekannten Methoden, z.B. wie hier und in den Beispielen beschrieben, vorgenommen, z.B. solvolytisch, etwa hydrolytisch oder acidolytisch wie oben beschrieben, oder bei veresterten Carboxygruppen auch reduktiv. So werden z.B. 2,2,2-Trichloräthyl- oder 2-Jodäthylester durch

Reduktion z.B. mit Zink und einer Carbonsäure in Gegenwart von Wasser in die Carbonsäure überführt. Benzylester oder Nitro-benzylester können durch katalytische Hydrierung oder auch durch Hydrierung mit chemischen Reduktionsmitteln, wie z.B. Natriumdithionit oder Zink und einer Carbonsäure, z.B. Essigsäure, in die Carboxygruppe umgewandelt werden. Ferner können tert.-Butylester z.B. auch mit Trifluoressigsäure gespalten werden.

Während der Reduktion des Restes C kann gleichzeitig eine Alkenylen- oder Alkinylenkette A in die entsprechende Alkylenkette umgewandelt werden.

Weiter können Verbindungen der Formel Ia, worin C Acetyl bedeutet, oxidativ zu den entsprechenden Verbindungen der Formel I, worin B Carboxy bedeutet, gespalten werden. Dazu wird die Acetylverbindung zunächst in eine Verbindung der Formel Ia, worin C Trihalogenacetyl, z.B. Tribrom- oder Trijodacetyl bedeutet, überführt, z.B. durch Behandlung mit Natrium-hypobromit, und nachfolgend z.B. mit einer wässerigen Base, etwa Natronlauge, gespalten.

Ausgangsstoffe der Formel Ia, in welchen C Acetyl bedeutet, können ausgehend von Verbindungen der Formel Ia, worin C Halogenmethyl bedeutet, durch Behandlung mit einem Acetessigsäure-niederalkylester, z.B. Acetessigsäure-äthylester, in Gegenwart einer Base, z.B. Natriumhydrid, und nachfolgende Hydrolyse mit einer starken Base, z.B. mit wässeriger Natronlauge, hergestellt werden.

Die genannten Verbindungen könnnen auch durch Kondensation einer Verbindung der Formel I, worin B Cyan bedeutet, z.B. mit einem Grignard- oder einem anderen metallorganischen Reagens, z.B. Methylmagnesium-bromid, unter Standardbedingungen hergestellt werden.

Ausgangsstoffe der Formel Ia, worin C Carboxycarbonyl (-COCOOH) bedeutet, können durch thermische Behandlung oder mittels Oxidation in Verbindungen der Formel I, worin B Carboxy bedeutet, überführt

werden. Der Ausgangsstoff wird dazu bei erhöhter Temperatur, z.B. ungefähr 200°C, in Gegenwart von Glaspulver erhitzt, oder z.B. mit Wasserstoffperoxid in Gegenwart einer Base, z.B. Natriumhydroxid, behandelt.

Die Ausgangsstoffe der Formel Ia, worin C Carboxycarbonyl bedeutet, können z.B. durch Kondensation einer Verbindung der Formel Ia, worin C Halogenmethyl bedeutet, z.B. mit 2-Aethoxycarbonyl-1,3-dithian, nachfolgende oxidative Hydrolyse, z.B. mit N-Bromsuccinimid in wässerigem Aceton, und schliesslich Behandlung mit z.B. verdünnter wässeriger Natronlauge erhalten werden.

Verbindungen der Formel Ia, worin C Formyl, Di-niederalkoxy-methyl oder Alkylendioxymethyl - letzteres entspricht als Acetal geschütztem Formyl -, z.B. Dimethylacetal, bedeutet, könnnen z.B. mit Silbernitrat, Pyridiniumdichromat oder Ozon zu den entsprechenden Verbindungen der Formel I, worin B Carboxy bedeutet, oxidiert werden.

Verbindungen der Formel Ia, worin C Vinyl bedeutet, können in Verbindungen der Formel I, worin B Carboxy ist, umgewandelt werden. Dazu werden sie zuerst durch Ozonolyse in Verbindungen der Formel Ia, worin C Formyl bedeutet, überführt, die ihrerseits in an sich bekannter Weise zu Verbindungen der Formel I, worin B Carboxy bedeutet, oxidiert werden können.

Ausgangsstoffe der Formel Ia, worin C Vinyl bedeutet, können auch mit Nickelcarbonyl und Kohlenmonoxid unter erhöhtem Druck behandelt werden, wobei man Verbindungen der Formel I, worin B Carboxy bedeutet und die Kette A in Nachbarschaft zur Carboxygruppe eine Doppelbindung trägt, erhält.

Verbindungen der Formel Ia, worin C Aethinyl bedeutet, können mit einer starken Base, z.B. Butyllithium, bei einer Temperatur von -70° bis +50°C behandelt, dann mit Kohlendioxid oder einem Halogenameisensäure-niederalkylester, z.B. Chlorameisensäureäthylester,

kondensiert und nachfolgend hydrolysiert werden. Man erhält Verbindungen der Formel I, worin B Carboxy bedeutet und die Kette A in Nachbarstellung zur Carboxygruppe eine Dreifachbindung enthält.

Verbindungen der Formel Ia, worin C Halogenmethyl bedeutet, können in die entsprechenden metallorganischen Zwischenprodukte, z.B. Kupfer- oder Magnesiumderivate, nach an sich bekannten Methoden umgewandelt werden.

Die Umsetzung z.B. eines erhaltenen organischen Magnesium-(Grignard)-Reagenses, z.B. einer Verbindung der Formel Ia, worin C in $CH_2MgCl$ umgewandelt ist, mit Kohlendioxid, ergibt eine Verbindung der Formel I, worin B Carboxy bedeutet und die Kette um ein Kohlenstoffatom verlängert ist.

Die Reaktion des genannten Grignard-Reagenses z.B. mit einem Halogenessigsäure-niederalkylester, z.B. Bromessigsäure-äthylester, und nachfolgende Hydrolyse ergibt eine Verbindung der Formel I, worin B Carboxy bedeutet und die Kette um 2 Kohlenstoffatome verlängert ist.

Das genannte Grignard-Reagens kann in Gegenwart eines Kupfer-I-halogenids, z.B. Kupfer-I-chlorids, mit einer α,ß-ungesättigten Säure, z.B. mit Propiolsäure oder Acrylsäure, kondensiert werden, wobei man eine Verbindung der Formel I erhält, worin B Carboxy bedeutet und die Kette durch 3 Kohlenstoffatome verlängert ist.

Ferner können Verbindungen der Formel Ia, worin C Halogenmethyl bedeutet, z.B. mit dem 3-Lithio-Derivat der Propiolsäure (hergestellt in situ aus Propiolsäure und z.B. Lithium-diisopropylamid) kondensiert werden, wobei man eine Verbindung der Formel I erhält, in welcher A terminales Alkinylen enthält, B für Carboxy steht und die Kette um 3 Kohlenstoffatome verlängert ist.

Verbindungen der Formel Ia, worin A Niederalkylen bedeutet und C für Hydroxymethyl oder insbesondere ein reaktionsfähiges funktionelles Derivat davon steht, können mit einem Niederalkanol (oder Niederalkanthiol), oder einem Phenol (oder Thiophenol), die jeweils in geeigneter Weise durch B substituiert sind, vorzugsweise in Gegenwart einer starken Base, umgesetzt werden. Man erhält Verbindungen der Formel I, worin A Niederalkylen-(thio oder oxy)-phenylen oder Niederalkylen-(thio oder oxy)-niederalkylen bedeutet.

Verbindungen der Formel Ia, worin A die oben angegebene Bedeutung hat, die Kettenlänge jedoch um mindestens 2 Kohlenstoffatome verkürzt ist, und C für Formyl steht oder Verbindungen der Formel Ia, worin A die oben angegebene Bedeutung hat, die Kettenlänge jedoch um mindestens 3 Kohlenstoffatome verkürzt ist, und C Acetyl bedeutet, können in Verbindungen der Formel I umgewandelt werden, worin A im Rahmen der oben angegebenen Bedeutung verlängert ist und Alkenylen, Niederalkylenphenylen-niederalkenylen oder Alkadienylen bedeutet und B wie oben definiert ist, indem man sie mit einer Verbindung der Formel XIa, $R_5$-A*-B, umsetzt, worin A* die oben für A angegebene Bedeutung hat, die Kettenlänge jedoch derart verkürzt ist, dass im Produkt der Formel I A im Rahmen seiner obigen Definition liegt. Diese Wittig-Reaktion wird unter den gleichen Bedingungen wie oben für Verfahren 7) angegeben durchgeführt.

Wenn irgendwelche der genannten Zwischenprodukte störende reaktionsfähige Gruppen, z.B. Carboxy-, Hydroxy- oder Aminogruppen, enthalten, können solche vorzugsweise vorübergehend, auf jeder Stufe, durch leichtabspaltbare Schutzgruppen geschützt werden. Der Zweck der Einführung von Schutzgruppen besteht darin, funktionelle Gruppen vor unerwünschten Reaktionen mit Reaktionspartnern zu bewahren und dadurch zu verhindern, dass sie abgespalten oder in Derivate umgewandelt werden. Auf der anderen Seite kann es geschehen, dass bei Anwesenheit ungeschützter funktioneller Gruppen Reaktionspartner in unerwünschter Weise durch Reaktion mit ersteren verbraucht oder gebunden werden und daher für die eigentlich vorgesehene Reaktion nicht zur Verfügung stehen. Die Wahl der Schutzgruppe für eine

bestimmte Reaktion hängt von verschiedenen Punkten ab, nämlich von der Art der zu schützenden funktionellen Gruppe, der Struktur und Stabilität des Moleküls, an dem die funktionelle Gruppe sitzt, und den Reaktionsbedingungen. Schutzgruppen, die diese Bedingungen erfüllen, ihre Einführung und Entfernung sind an sich bekannt und beschrieben, z.B. in J.F.W. McOmie, "Protective Groups in Organic Chemistry", Plenum Press, London, New York 1973. So können Carboxygruppen z.B. als Ester geschützt werden, z.B. als unsubstituierte oder substituierte Niederalkylester, wie etwa dem Methyl- oder Benzylester, wobei es möglich ist, solche Estergruppen unter milden Bedingungen, insbesondere alkalischen Bedingungen, wieder leicht abzuspalten. Amino- oder Hydroxy-Schutzgruppen, die unter schonenden Bedingungen wieder abgespalten werden können, sind z.B. Acylradikale, wie etwa gegebenenfalls durch Halogen substituiertes Niederalkanoyl, z.B. Formyl oder Trichloracetyl, oder organische Silylgruppen, z.B. Triniederalkylsilyl, wie etwa Trimethylsilyl. In einer Verbindung der Formel I oder in irgendeinem Zwischenprodukt, die eine oder mehrere funktionelle Gruppen in geschützter Form enthalten, z.B. geschütztes Carboxy, Amino oder Hydroxy, können letztere in an sich bekannter Weise z.B. durch Solvolyse, etwa Hydrolyse, freigesetzt werden.

Die oben genannten Reaktionen werden nach an sich bekannten Methoden, in Gegenwart oder Abwesenheit von Verdünnungsmitteln, vorzugsweise in solchen, die gegenüber den Reagenzien inert sind und diese lösen, Katalysatoren, Kondensations- oder anderen obengenannten Mitteln, und/oder einer inerten Atmosphäre, unter Kühlung, bei Zimmertemperatur oder bei erhöhter Temperatur, vorzugsweise beim Siedepunkt des verwendeten Lösungsmittels, bei normalem oder erhöhtem Druck, durchgeführt. Die bevorzugten Lösungsmittel, Katalysatoren und Reaktionsbedingungen sind in den beigefügten Beispielen offenbart.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die

verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin einer oder mehrere der Ausgangsstoffe in Form eines Salzes oder eines optisch reinen Antipoden verwendet wird.

Im Verfahren der vorliegenden Erfindung werden bevorzugt solche Ausgangsstoffe verwendet, die zu den im Vorstehenden als besonders wertvoll beschriebenen Verbindungen führen.

Die Erfindung betrifft auch neue Ausgangsstoffe und Verfahren zu ihrer Herstellung.

Abhängig von der Wahl der Ausgangsstoffe und dem verwendeten Verfahren können die neuen Verbindungen in Form eines der möglichen Isomeren oder als Isomerengemische vorliegen. So können sie, abhängig von der Gegenwart einer Doppelbindung und von der Anzahl der asymmetrischen Kohlenstoffatome, z.B. reine optische Isomeren, etwa Antipoden, oder Gemische von optischen Isomeren, z.B. Racemate, Gemische von Diastereomeren, Gemische von Racematen oder Gemische von geometrischen Isomeren sein. Die vorher genannten möglichen Isomeren und ihre Gemische gehören zum Umfang der vorliegenden Erfindung. Bestimmte spezifische Isomeren können bevorzugt sein.

Erhaltene Gemische von Diastereomeren, Gemische von Racematen oder geometrischen Isomeren können auf der Basis der physikochemischen Unterschiede ihrer Komponenten in an sich bekannter Weise in die reinen Isomeren, Diasteromeren, Racemate oder geometrischen Isomeren, z.B. durch Chromatographie und/oder fraktionierte Kristallisation, getrennt werden.

Erhaltene Racemate können weiter in die optischen Antipoden in an sich bekannter Weise, z.B. durch Umsetzung eines sauren Endproduktes mit einer optisch aktiven Base, die mit der racemischen Säure Salze bildet, aufgetrennt werden. Diese Salze können z.B. durch fraktionierte Kristallisation in die diastereomeren Salze getrennt

werden. Aus letzteren können die optisch aktiven Säure-Antipoden durch Ansäuren freigesetzt werden. Basische racemische Produkte können in analoger Weise, z.B. durch Trennung ihrer diastereomeren Salze mit einer optisch aktiven Säure und Freisetzung der optisch aktiven basischen Endprodukte mit einer gewöhnlichen Base, getrennt werden. Racemische Produkte der Erfindung können auf diese Weise in ihre optische Antipoden gespalten werden, z.B. durch fraktionierte Kristallilsation von d- oder l-(Tartraten, Mandelaten, Camphersulfonaten, oder von d- oder l-(α-Methylbenzylamin, Cinchonidin, Cinchonin, Chinin, Chinidin, Ephedrin, Dehydroabietylamin, Brucin oder Strychin)-salzen. Vorzugsweise wird von zwei Antipoden der stärker wirksame isoliert.

Schliesslich können die Verbindungen der Erfindung in freier Form oder als Salze erhalten werden. Eine erhaltene freie Base kann in das entsprechende Säureadditionssalz vorzugsweise mit Säuren, die therapeutisch verwendbare Säureadditionssalze ergeben, oder mit Anionenaustauschern überführt werden. Erhaltene Salze können in die entsprechenden freien Basen z.B. durch Behandlung mit einer stärkeren Base, wie einem Metall- oder Ammoniumhydroxid oder einem basischen Salz, z.B. einem Alkalimetallhydroxid oder -carbonat, oder mit einem Kationenaustauscher umgewandelt werden. Eine Verbindung der Formel I, in welcher B Carboxy bedeutet, kann auf diese Weise auch in die entsprechenden Metall- oder Ammoniumsalze überführt werden. Diese oder andere Salze, z.B. die Pikrate, können auch in der Reinigung von erhaltenen freien Basen verwendet werden. Die Basen werden in ihre Salze überführt, die Salze abgetrennt und die Basen aus den Salzen freigesetzt.

Infolge der engen Beziehung zwischen den neuen Verbindungen in freier Form und in Form ihrer Salze sind im Vorausgegangenen und nachfolgend unter freien Verbindungen und Salzen sinn- und zweckgemäss gegebenenfalls auch die entsprechenden Salze bzw. freien Verbindungen zu verstehen.

Die Verbindungen und ihre Salze können auch in Form ihrer Hydrate erhalten werden oder andere, für die Kristallisation verwendete Lösungsmittel einschliessen.

Die erfindungsgemässen pharmazeutischen Präparate sind solche, die sich für enterale, z.B. orale oder rektale, und parenterale Verabreichung an Säuger, einschliesslich Menschen, eignen. Diese Präparate enthalten eine wirksame Dosis wenigstens einer pharmakologisch aktiven Verbindung der Formel I, oder eines ihrer pharmazeutisch verwendbaren Salze, allein oder in Kombination mit einem oder mehreren pharmazeutisch annehmbaren Trägermaterialien.

Die pharmakologisch wirksamen Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z.B. Laktose, Dextrose, Rohrzucker, Mannitol, Sorbitol, Cellulose und/oder Glycin, und Schmiermitteln, z.B. Kieselerde, Talk, Stearinsäure oder ihren Salzen, wie Magnesium- oder Calciumstearat, und/oder Polyäthylenglykol, enthalten; Tabletten enthalten ebenfalls Bindemittel, z.B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen und/oder Adsorptionsmittel, Farbstoffe, Geschmackstoffe und Süssmittel. Injizierbare Präparate sind vorzugsweise isotonische wässerige Lösungen oder Suspensionen, und Suppositorien in erster Linie Fettemulsionen oder -suspensionen. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten

können, werden in an sich bekannter Weise, z.B. mittels konventioneller Misch-, Granulier- oder Dragierverfahren, hergestellt und enthalten etwa 0,1 % bis etwa 75 %, insbesondere etwa 1 % bis etwa 50 %, des Aktivstoffes. Einzeldosen für Säuger mit einem Gewicht von ungefähr 50 bis 70 kg können ungefähr 10 bis 100 mg des aktiven Bestandteils enthalten.

Die folgenden Beispiele dienen zur Illustration der Erfindung und sind nicht als Einschränkung ihres Umfangs aufzufassen. Temperaturen werden in Celsiusgraden angegeben, und Angaben über Teile betreffen Gewichtsteile. Wenn nicht anders definiert, wird das Eindampfen von Lösungsmitteln unter vermindertem Druck, vorzugsweise zwischen ungefähr 20 und 130 mbar, durchgeführt.

Beispiel 1: Eine Lösung von 1,27 ml Diisopropylamin in 25 ml Tetrahydrofuran wird auf -65° unter Stickstoff abgekühlt und mit 5,7 ml 1,6 m n-Butyllithium in Hexan versetzt. Nach 30 min bei -65° werden 2,0 ml 4-Phosphoncrotonsäure-triäthylester tropfenweise zugegeben. Das Reaktionsgemisch wird 30 min gerührt, bevor eine Lösung von 2,0 g N-(3-Pyridyl)-indol-3-carboxaldehyd in 20 ml Tetrahydrofuran langsam zugegeben wird, wobei die Temperatur auf unter -60° gehalten wird. Nach beendeter Zugabe wird die Kühlung unterbrochen, und das Reaktionsgemisch erwärmt sich langsam auf Raumtemperatur, bei der es vier Stunden gerührt wird. Das Lösungsmittel wird abgedampft und der Rückstand zwischen 30 ml Methylenchlorid und 20 ml halb-gesättigter Ammoniumchloridlösung verteilt. Die organische Phase wird mit 20 ml Wasser gewaschen, über Natriumsulfat getrocknet und zu einem Oel eingedampft, das aus heissem Aethylacetat kristallisiert. Man erhält 3-[4-(Aethoxycarbonyl)-1,3-butadienyl]-N-(3-pyridyl)-indol, Fp. 112 bis 113°.

Die Ausgangsverbindungen werden wie folgt hergestellt:
Eine Lösung von 2,12 ml N,N-Dimethylformamid in 20 ml 1,2-Dichloräthan wird auf 0° gekühlt und mit 3,1 ml Phosphoroxychlorid tropfenweise versetzt. Das Reaktionsgemisch wird 10 min bei 15° gerührt und dann wieder auf 0° abgekühlt, bevor eine Lösung von 4,85 g

N-(3-Pyridyl)-indol in 10 ml 1,2-Dichloräthan zugegeben wird. Nach 2 h bei 5° wird das Reaktionsgemisch 15 min unter Rückfluss erhitzt, auf Raumtemperatur abgekühlt und mit einer Lösung von 18,75 g Natriumacetat-Trihydrat in 25 ml Wasser versetzt. Nach weiteren 15 min Rückfluss wird die organische Phase abgetrennt, mit Natriumhydrogencarbonat und Wasser gewaschen und über Magnesiumsulfat getrocknet. Nach dem Filtrieren, Eindampfen und Umkristallisieren des Rückstands aus Methylenchlorid/Methanol erhält man N-(3-Pyridyl)-indol-3-carboxaldehyd, Fp. 148 bis 150°.

N-(3-Pyridyl)-indol wird gemäss J. Chem. Soc. (C) 85 (1970) hergestellt.

Beispiel 2: Eine Lösung von 6,0 g 3-[(4-Aethoxycarbonyl)-1,3-butadienyl]-N-(3-pyridyl)-indol in 60 ml 95-proz. Aethanol wird hydriert, und zwar bei einem Druck von 3 bar mit 0,6 g 10 % Palladium-auf-Kohle, bis die theoretische Menge an Wasserstoff aufgenommen ist. Die Lösung wird durch Cellit filtriert und eingedampft; man erhält 3-[4-(Aethoxycarbonyl)-butyl]-N-(3-pyridyl)-indol; IR (Nujol) 1720 $cm^{-1}$.

Beispiel 3: Ein Gemisch von 6,8 g 3-[(4-Aethoxycarbonyl)-butyl]-N-(3-pyridyl)-indol und 1,68 g Natriumhydroxid in 30 ml Wasser und 30 ml Methanol wird 30 min unter Rückfluss erhitzt, 4 h bei Raumtemperatur gerührt und zu einem Sirup konzentriert, der in 30 ml Wasser bei 5° wieder aufgelöst wird. Die Lösung wird mit 3,5 ml konzentrierter Salzsäure neutralisiert. Das entstandene Oel kristallisiert aus, wird abfiltriert und getrocknet; man erhält 3-(4-Carboxybutyl)-N-(3-pyridyl)-indol, Fp. 122 bis 124°.

Beispiel 4: Eine Lösung von 8,0 g 3-(3-Indolyl)-propionsäure-äthylester und 6,3 g 3-Brompyridin in 100 ml Pyridin wird mit 5,0 g Kupferoxid und 10 g wasserfreiem Kaliumcarbonat 48 h unter Rückfluss erhitzt. Nach dem Eindampfen und der Chromatographie auf 300 g

Kieselgel mit 1:1 Diäthyläther/Hexan als Elutionsmittel erhält man 3-[2-(Aethoxycarbonyl)-äthyl]-N-(3-pyridyl)-indol als Oel; $R_f$ = 0,2, IR ($CH_2Cl_2$) $1725^{-1}$.

Die Ausgangsverbindung wird wie folgt hergestellt:
Eine Lösung von 7,38 g 3-Indolpropionsäure und 5,9 g Thionylchlorid in 50 ml Aethanol wird 2 h unter Rückfluss erhitzt, abgekühlt und eingedampft. Der Rückstand wird zwischen Aether und kalter Natriumhydrogencarbonatlösung verteilt. Die organische Phase wird abgetrennt, über Natriumsulfat getrocknet und eingedampft; man erhält 3-(3-Indolyl)-propionsäure-äthylester; $R_f$ (Aether/$SiO_2$) = 0,51; IR ($CH_2Cl_2$) 1725 $cm^{-1}$.

Beispiel 5: Eine Lösung von 2,0 g 3-[2-(Aethoxycarbonyl)-äthyl]-N-(3-pyridyl)-indol in 10 ml Methanol und 10 ml 1 n Natronlauge wird 2 h unter Rückfluss erhitzt, abgekühlt und eingedampft. Der Rückstand wird in 10 ml Wasser aufgenommen, mit 10 ml Aether extrahiert und auf pH 6 mit konzentrierter Schwefelsäure eingestellt. Der entstandene Feststoff wird abfiltriert und getrocknet; man erhält 3-(2-Carboxyäthyl)-N-(3-pyridyl)-indol, Fp. 147 bis 149°.

Beispiel 6: Eine Lösung von 2,7 g 3-[2-(Aethoxycarbonyl)-äthyl]-N-(3-pyridyl)-indol in 50 ml trockenem Methylenchlorid wird unter Stickstoffschutz auf -75° abgekühlt und mit 10,6 ml 1,75 m Diisobutylaluminiumhydrid in Toluol tropfenweise versetzt. Das Reaktionsgemisch wird 8 min gerührt, dann die Reaktion durch Zugabe von 10 ml Wasser bei -75° beendet. Die Kühlung wird aufgegeben, und die Phasen werden getrennt. Die organische Phase wird mit Wasser gewaschen, über Kaliumcarbonat getrocknet und zu einem Feststoff eingedampft, der aus Aether/Hexan umkristallisiert wird. Man erhält 3-(2-Formyläthyl)-N-(3-pyridyl)-indol, Fp. 44 bis 45°.

Beispiel 7: Eine Lösung von Lithiumdiisopropylamid (aus 0,53 g Diisopropylamin in 30 ml Tetrahydrofuran und 3,15 ml 1,65 m n-Butyllithium) wird auf -75° unter Stickstoffschutz abgekühlt und mit 1,19 g 2-Phosphonpropionsäure-triäthylester tropfenweise versetzt.

Das Reaktionsgemisch wird 45 min gerührt und mit einer Lösung von 0,90 g 3-(2-Formyläthyl)-N-(3-pyridyl)-indol in 10 ml Tetrahydrofuran versetzt. Man rührt das Reaktionsgemisch 15 min bei -75°, lässt es im Verlauf von 45 min auf Raumtemperatur erwärmen, beendet die Reaktion durch Zugabe von überschüssiger Ammoniumchloridlösung und verdünnt mit 30 ml Diäthyläther. Die organische Phase wird abgetrennt, über Kaliumcarbonat getrocknet und eingedampft; man erhält ein Gemisch von (E)- und (Z)-3-[4-(Aethoxycarbonyl)-pent-3-enyl]-N-(3-pyridyl)-indol als Oel; $R_f$ ($SiO_2$/2:1 Aether/Hexan) = 0,33 bzw. 0,40.

Beispiel 8: Eine Lösung von 1,4 g (Z)- und (E)-3-[4-(Aethoxycarbonyl)-pent-3-enyl]-N-(3-pyridyl)-indol in 10 ml Methanol und 6,0 ml 1 n Natronlauge wird 3 h unter Rückfluss erhitzt, abgekühlt und eingedampft. Der Rückstand wird in 10 ml Wasser wieder aufgelöst, die Lösung wird auf pH 6 mit konzentrierter Schwefelsäure eingestellt und mit Chloroform extrahiert. Die organische Phase wird über Natriumsulfat getrocknet und eingedampft. Das anfallende Oel wird in Aceton wieder aufgelöst und mit 1,13 ml 3 n ätherischem Chlorwasserstoff tropfenweise versetzt. Das entstandene Salz wird abfiltriert; man erhält (E)-3-(4-Carboxypent-3-enyl)-N-(3-pyridyl)-indol-Hydrochlorid, Fp. 210 bis 211°.

Die Mutterlaugen werden eingedampft, und der Rückstand wird aus Aceton umkristallisiert; man erhält (Z)-3-(4-Carboxypent-3-enyl)-N-(3-pyridyl)-indol-Hydrochlorid, Fp. 134 bis 135°.

Beispiel 9: Eine Lösung von 2,5 g 3-(3-Indolyl)-buttersäure-äthylester und 1,15 ml 3-Brompyridin in 25 ml Pyridin wird mit 1,25 g Kupferoxid und 1,25 g Kaliumcarbonat 72 h unter Rückfluss erhitzt, filtriert und zu einem braunen Oel eingedampft, das auf 64 g Kieselgel mit 8:1 Toluol/Aethylacetat chromatographiert wird. Man erhält 3-(3-Aethoxycarbonyl-2-propyl)-N-(3-pyridyl)-indol, $R_f$ (15 % Aethylacetat, 85 % Toluol/Kieselgel) = 0,35; IR ($CH_2Cl_2$) 1725 $cm^{-1}$.

3-(3-Indolyl)-buttersäure-äthylester wird gemäss Chem. Pharm. Bull. 30, 3092 (1982) hergestellt.

Beispiel 10: Eine Lösung von 2,66 g 3-(3-Aethoxycarbonyl-2-propyl)-N-(3-pyridyl)-indol in 15 ml Methanol und 15 ml Wasser, das 0,7 g Natriumhydroxid enthält, wird bei Raumtemperatur 18 h gerührt, 15 min unter Rückfluss erhitzt, abgekühlt und eingedampft. Der Rückstand wird in 25 ml Wasser wieder aufgelöst und auf pH 6 mit 1,44 ml konzentrierter Salzsäure neutralisiert. Das entstandene Oel wird in Methylenchlorid extrahiert und der Extrakt über Natriumsulfat getrocknet. Durch Filtrieren und Eindampfen erhält man ein Oel, das in 5 ml Tetrahydrofuran wieder aufgelöst und mit 0,55 ml konzentrierter Salzsäure behandelt wird. Der entstandene Halbfeststoff wird aus Methanol/Aether kristallisiert; man erhält 3-(3-Carboxy-2-propyl)-N-(3-pyridyl)-indol-Hydrochlorid, Fp. 205 bis 207°.

Beispiel 11: Eine Lösung von 1,0 g 3-(2-Formyläthyl)-N-(3-pyridyl)-indol in 15 ml Methylenchlorid wird unter Stickstoff auf -20° abgekühlt und mit 4,6 ml 1,75 m Diisobutylaluminiumhydrid in Toluol tropfenweise versetzt. Man lässt das Reaktionsgemisch auf Raumtemperatur innerhalb von 2 h erwärmen und gibt 20 ml Wasser unter starkem Rühren zu. Das Reaktionsgemisch wird durch Cellit filtriert, wobei mit Methylenchlorid weiter gewaschen wird. Die organische Phase wird abgetrennt, über Kaliumcarbonat getrocknet und eingedampft; man erhält 3-(3-Hydroxypropyl)-N-(3-pyridyl)-indol als Oel; IR ($CH_2Cl_2$) 3480 $cm^{-1}$.

Beispiel 12: Eine Lösung von 0,36 g Mercaptoessigsäure-äthylester in 20 ml Dimethylformamid wird mit 0,145 g einer 50 %igen Oeldispersion von Natriumhydrid bei Raumtemperatur unter Stickstoff während 15 min behandelt. Eine Lösung von 1,13 g 3-(3-Phenylsulfonyloxy-propyl)-N-(3-pyridyl)-indol in 5 ml Dimethylformamid wird bei Raumtemperatur zugegeben. Das Reaktionsgemisch wird 15 h gerührt, mit 100 ml Wasser verdünnt und viermal mit je 50 ml Diäthyläther extrahiert. Die organischen Extrakte werden mit 50 ml Wasser und 20 ml kalter 1 n

Salzsäure gewaschen. Die Säurephase wird mit Aether extrahiert und auf pH 8,5 mit 50 %iger Natronlauge gebracht. Die wässerige Phase wird mit Chloroform extrahiert (3 x 30 ml). Der Chloroformextrakt wird über Kaliumcarbonat getrocknet und eingedampft; man erhält 3-[3-(Aethoxycarbonylmethylthio)-propyl]-N-(3-pyridyl)-indol; IR ($CH_2Cl_2$) 1725 $cm^{-1}$.

Die Ausgangsverbindung wird wie folgt hergestellt:
Eine Lösung von 1,0 g 3-(3-Hydroxypropyl)-N-(3-pyridyl)-indol in 40 ml trockenem Pyridin wird bei 0° mit 0,7 g Benzolsulfonylchlorid behandelt und 14 h bei 0° stehengelassen. Das Lösungsmittel wird abgedampft und der Rückstand zwischen Diäthyläther und wässeriger Natriumhydrogencarbonatlösung verteilt. Die organische Phase wird abgetrennt, über Kaliumcarbonat getrocknet und eingedampft; man erhält rohes 3-(3-Phenylsulfonyloxypropyl)-N-(3-pyridyl)-indol als Oel, das ohne weitere Reinigung verwendet wird.

Beispiel 13: Eine Lösung von 90 mg 3-[3-(Aethoxycarbonylmethylthio)-propyl]-N-(3-pyridyl)-indol in 5 ml Methanol und 1 ml 1 n Natronlauge wird 10 h unter Rückfluss erhitzt, abgekühlt und eingedampft. Das entstandene Oel wird zwischen Aether und Wasser verteilt. Die wässerige Phase wird auf pH 6 mit konzentrierter Schwefelsäure gebracht und mit Chloroform extrahiert; der Chloroformextrakt wird über Natriumsulfat getrocknet. Filtrieren und Eindampfen gibt ein Oel, das in 2 ml Aceton wieder aufgelöst und mit 0,07 ml 3 n ätherischem Chlorwasserstoff behandelt wird. Nach dem Eindampfen und Kristallisieren aus Aceton/Aether erhält man 3-[3-(Carboxymethylthio)-propyl]-N-(3-pyridyl)-indol-Hydrochlorid, Fp. 106 bis 110°.

Beispiel 14: Eine Lösung von Dimsyllithium (aus 80 ml Dimethylsulfoxid und 13 ml 2,1 m n-Butyllithium) wird unter Stickstoff bei Raumtemperatur gerührt und mit 6,2 g 5-Carboxypentyltriphenylphosphoniumbromid portionsweise versetzt. Das Reaktionsgemisch wird 30 min gerührt und mit 3,0 g N-(3-Pyridyl)-indol-3-carboxaldehyd versetzt. Nach 18 h bei Raumtemperatur wird das Reaktionsgemisch mit 240 ml Wasser verdünnt und mit 100 ml Aethylacetat extrahiert. Die

wässerige Phase wird mit 1,1 ml konzentrierter Salzsäure neutralisiert und mit Aethylacetat extrahiert. Die Extrakte weren über Natriumsulfat getrocknet und zu einem Oel eingedampft, das mit Aether zu einem Halbfeststoff verrieben wird, der aus Methanol umkristallisiert wird; man erhält 3-(6-Carboxyhex-1-enyl)-N-(3-pyridyl)-indol, Fp. 124 bis 125°.

Beispiel 15: Eine Lösung von 0,5 g 3-(6-Carboxyhex-1-enyl)-N-(3-pyridyl)-indol in 25 ml 95 %igem Aethanol wird hydriert, und zwar bei einem Druck von 3 bar mit 0,1 g 10 % Palladium-auf-Kohle während 17 h, dann durch Cellit filtriert und zu einem Oel eingedampft. Durch Kristallisation aus Methanol erhält man 3-(6-Carboxyhexyl)-N-(3-pyridyl)-indol, Fp. 120 bis 122°.

Beispiel 16: Eine Lösung von Lithiumdiisopropylamid (aus 1,4 ml Diisopropylamin und 6,3 ml 1,6 m n-Butyllithium in Hexan) in 25 ml trockenem Tetrahydrofuran wird unter Stickstoff auf -70° abgekühlt und mit 2,14 ml 2-Phosphonpropionsäure-triäthylester tropfenweise versetzt. Das Reaktionsgemisch wird 20 min bei -70° gerührt und mit 2,0 g N-(3-Pyridyl)-indol-3-carboxaldehyd versetzt. Man lässt das Reaktionsgemisch im Verlauf von 14 h auf Raumtemperatur erwärmen. Das Lösungsmittel wird abgedampft und der Rückstand zwischen 20 ml halbgesättigter Ammoniumchloridlösung und 30 ml Methylenchlorid verteilt. Die organische Phase wird abgetrennt, über Natriumsulfat getrocknet und zu einem Feststoff eingedampft, der aus Methanol umkristallisiert wird; man erhält 3-[2-(Aethoxycarbonyl)-prop-1-enyl]-N-(3-pyridyl)-indol, Fp. 97 bis 99°.

Beispiel 17: Eine Lösung von 1,1 g 3-[2-(Aethoxycarbonyl)-prop-1-enyl]-N-(3-pyridyl)-indol und 0,288 g Natriumhydroxid in 10 ml Methanol und 10 ml Wasser wird 2 h unter Rückfluss erhitzt, abgekühlt und eingedampft. Das entstandene Oel wird in 10 ml Eiswasser wieder gelöst und mit 0,59 ml konzentrierter Salzsäure neutralisiert. Der entstandene Feststoff wird filtriert und getrocknet; man erhält 3-(2-Carboxyprop-1-enyl)-N-(3-pyridyl)-indol, Fp. 213 bis 215°.

<u>Beispiel 18</u>: Eine Lösung von Lithiumdiisopropylamid (aus 0,57 ml Diisopropylamin und 2,5 ml 1,6 m n-Butyllithium in Hexan) in 20 ml trockenem Tetrahydrofuran wird unter Stickstoff auf -70° abgekühlt und mit 0,78 ml 4-Phosphoncrotonsäure-triäthylester tropfenweise versetzt. Das Reaktionsgemisch wird 30 min bei -70° gerührt, dann mit einer Lösung von 0,9 g N-(3-Pyridyl)-indol-2-carboxaldehyd in 5 ml Tetrahydrofuran langsam versetzt. Man lässt das Reaktionsgemisch auf Raumtemperatur erwärmen, rührt es 14 h und dampft es zu einem Oel ein, das zwischen 15 ml halbgesättigter Ammoniumchloridlösung und 40 ml Diäthyläther verteilt wird. Die organische Phase wird mit 10 ml Wasser gewaschen, über Natriumsulfat getrocknet und eingedampft; man erhält 2-[4-(Aethoxycarbonyl)-1,3-butadienyl]-N-(3-pyridyl)-indol als wachsartigen Feststoff; $R_f$ (10 % Methanol in Toluol, Kieselgel) = 0,7.

Die Ausgangsverbindung wird wie folgt hergestellt:
Eine Lösung von 10,0 g 2-Aethoxycarbonylindol und 5 ml 3-Brompyridin in 50 ml Pyridin wird mit 5,0 g Kupferoxid und 5,0 g Kaliumcarbonat 72 h unter Rückfluss erhitzt. Filtrieren und Eindampfen gibt ein Oel, das auf 110 g Kieselgel mit 15 % Aethylacetat in Toluol chromatographiert wird; man erhält 2-Aethoxycarbonyl-N-(3-pyridyl)-indol als Oel; IR ($CH_2Cl_2$) 1705 $cm^{-1}$.

Eine Lösung von 5,0 g 2-Aethoxycarbonyl-N-(3-pyridyl)-indol in 75 ml trockenem Aether wird auf 5° abgekühlt und mit 0,7 g Lithiumaluminiumhydrid portionsweise versetzt. Die Kühlung wird unterbrochen, das Reaktionsgemisch wird bei Raumtemperatur unter Stickstoff 18 h gerührt und wieder auf 5° abgekühlt. Die anschliessende Zugabe von 0,7 ml Wasser, 0,7 ml 15 %iger Natronlauge und 2,1 ml Wasser gibt einen feinen Niederschlag, der durch Filtrieren durch Cellit entfernt wird. Das Filtrat wird über Natriumsulfat getrocknet und eingedampft; man erhält rohes 2-Hydroxymethyl-N-(3-pyridyl)-indol, das ohne weitere Reinigung verwendet wird; $R_f$ (9:1 Toluol/Methanol, Kieselgel) = 0,2.

Eine Lösung von 3,3 g 2-Hydroxymethyl-N-(3-pyridyl)-indol in 33 ml trockenem Toluol wird unter Stickstoff mit 16,5 g Silbercarbonat 24 h unter Rückfluss erhitzt. Filtrieren durch Cellit und Eindampfen gibt ein Oel, das durch präparative Schichtchromatographie auf Kieselgel mit Methylenchlorid/Aethylacetat (8:2) als Elutionsmittel gereinigt wird; man erhält N-(3-Pyridyl)-indol-2-carboxaldehyd, Fp. 101 bis 103°.

Beispiel 19: Eine Lösung von 1,1 g 2-[4-(Aethoxycarbonyl)-1,3-butadienyl]-N-(3-pyridyl)-indol in 25 ml 95 %igem Aethanol wird hydriert, und zwar bei einem Druck von 3 bar mit 0,2 g 10 % Palladium-auf-Kohle während 3 1/2 h. Das Reaktionsgemisch wird durch Cellit filtriert und eingedampft; man erhält 2-[4-(Aethoxycarbonyl)-butyl]-N-(3-pyridyl)-indol als gelbes Oel; $R_f$ (15 % Aethylacetat in Toluol/Kieselgel) = 0,65; IR ($CH_2Cl_2$) 1726 $cm^{-1}$.

Beispiel 20: Eine Lösung von 1,0 g 2-[4-(Aethoxycarbonyl)-butyl]-N-(3-pyridyl)-indol und 0,25 g Natriumhydroxid in 5 ml Methanol und 5 ml Wasser wird 17 h bei Raumtemperatur gerührt. Das Lösungsmittel wird abgedampft, der Rückstand wird in 10 ml Wasser wieder aufgelöst und mit 0,515 ml konzentrierter Salzsäure neutralisiert. Das entstandene Produkt wird aus Methanol umkristallisiert; man erhält 2-(4-Carboxybutyl)-N-(3-pyridyl)-indol, Fp. 143 bis 144°.

Beispiel 21: Die folgenden Verbindungen werden analog zu den in den vorhergehenden Beispielen beschriebenen Methoden hergestellt.

| Verbindung | Name |
|---|---|
| 1 | 3-(3-Carboxypropyl)-N-(3-pyridyl)-indol, Fp. 134 bis 135°. |
| 2 | 5-Brom-3-(4-carboxybutyl)-N-(3-pyridyl)-indol, Fp. 118 bis 120°. |
| 3 | 3-(4-Carboxybutyl)-2-methyl-N-(3-pyridyl)-indol, Fp. 112 bis 114° |
| 4 | 3-(4-Carboxybutyl)-7-methyl-N-(3-pyridyl)-indol, Fp. 118 bis 119°. |

| | |
|---|---|
| 5 | 3-(4-Carboxybutyl)-5-methoxy-N-(3-pyridyl)-indol, Fp. 100°. |
| 6 | 3-(4-Carboxybutyl)-5-chlor-N-(3-pyridyl)-indol, Fp. 120 bis 122°. |
| 7 | 3-(4-Carboxybutyl)-5-methyl-N-(3-pyridyl)-indol, Fp. 76 bis 77°. |
| 8 | 3-(4-Carboxy-1,3-butadienyl)-5-methoxy-N-(3-pyridyl)-indol, Fp. 215 bis 217°. |
| 9 | 3-(4-Aethoxycarbonylbutyl)-5-methoxy-N-(3-pyridyl)-indol-Hydrochlorid, Fp. 108 bis 110°. |
| 10 | 3-(5-Carboxypent-1-enyl)-N-(3-pyridyl)-indol, Fp. 146 bis 148°. |
| 11 | 3-(5-Carboxypentyl)-N-(3-pyridyl)-indol; Fp. 132 bis 133°. |
| 12 | 3-(6-Carboxyhex-2-enyl)-6-chlor-N-(3-pyridyl)-indol-Hydrochlorid-Monohydrat, Fp. 156 bis 158°. |
| 13 | 3-Carboxymethyl-6-chlor-N-(3-pyridyl)-indol, Fp. 224-226°. |
| 14 | 3-(6-Carboxyhexyl)-5-chlor-N-(3-pyridyl)-indol, Fp. 126-127°. |
| 15 | 3-(6-Carboxy-1-hexenyl)-5-chlor-N-(3-pyridyl)-indol, Fp. 143-147°. |

Für die Herstellung der Verbindungen 2 bis 9 und 12 werden als Ausgangsverbindungen entweder 5-Brom-, 2-Methyl-, 7-Methyl-, 5-Methoxy-, 5-Chlor-, 5-Methyl- oder 6-Chlorindol verwendet, die z.B. zuerst in das entsprechende N-(3-Pyridyl)indol gemäss J. Chem. Soc. (C) 85 (1970) umgewandelt werden.

Die Verbindung 10 wird z.B. aus N-(3-Pyridyl)-indol-3-carboxaldehyd und 4-Carboxybutyl-triphenylphosphonium-bromid gemäss Beispiel 14 hergestellt.

Die Verbindung 1 wird z.B. durch Behandeln von 3-Indolbuttersäure-äthylester mit 3-Brompyridin und anschliessende Hydrolyse gemäss den vorhergehenden Beispielen hergestellt.

Beispiel 22: Eine Suspension von 26,6 g 3-(4-Carboxybutyl)-N-(3-pyridyl)-indol und 10,5 g Sulfamid in 90 ml Sulfolan wird mit 11,42 g Thionylchlorid bei Raumtemperatur unter Stickstoffschutz behandelt. Das Gemisch wird auf 120° erhitzt, bis die Freisetzung von Gas aufhört; dann wird festes p-Toluolsulfonsäure-Monohydrat (1,71 g) vorsichtig zugegeben. Nach dem Erhitzen während drei weiterer Stunden bei 120° wird das Reaktionsgemisch abgekühlt, auf 200 g Eis gegossen und mit 130 ml 1 n Salzsäure angesäuert. Die wässerige Phase wird dann mit Aethylacetat extrahiert (3 x 100 ml), mit festem Natriumhydrogencarbonat alkalisch gemacht und wieder mit Aethylacetat extrahiert (3 x 125 ml). Die organischen Extrakte werden mit 0,5 n Natronlauge (5 x 50 ml) gewaschen, über Natriumsulfat getrocknet und eingedampft; man erhält ein Oel, das auf 60 g Kieselgel chromatographiert wird; man erhält 3-(4-Cyanbutyl)-N-(3-pyridyl)-indol.

Beispiel 23: Ein Gemisch von 5,16 g 3-(4-Cyanbutyl)-N-(3-pyridyl)-indol, 1,88 g Natriumazid, 1,57 g Ammoniumchlorid und 10 Mol-% Lithiumchlorid (0,12 g) wird in 14 ml trockenem N,N-Dimethylformamid bei 125° 17 h erhitzt. Das Reaktionsgemisch wird abgekühlt, filtriert und zu einem Oel eingedampft, das zwischen 50 ml Wasser und 50 ml Aethylacetat verteilt wird. Die wässerige Phase wird auf pH 2 eingestellt, mit 20 ml Aethylacetat extrahiert und auf pH 5 eingestellt; der entstandene Feststoff wird durch Filtrieren gesammelt. Nach der Behandlung mit 6,2 ml 3 n ätherischem Chlorwasserstoff erhält man 3-[4-(5-Tetrazolyl)-butyl]-N-(3-pyridyl)-indol-Hydrochlorid.

Beispiel 24: Eine Lösung von Hydroxylamin (aus 2,06 g Hydroxylamin-Hydrochlorid und 2,02 g Natriumhydroxid) und 7,6 g 3-(4-Methoxycarbonylbutyl)-N-(3-pyridyl)-indol in 25 ml Methanol wird bei Raumtemperatur 20 h stehengelassen. Das Methanol wird abgedampft, der Rückstand wird in 5 ml Wasser aufgenommen und auf pH 7 eingestellt. Das Gemisch wird mit Methylenchlorid extrahiert; der Extrakt wird mit Wasser gewaschen, getrocknet und zur Trockene eingedampft; man erhält 3-[4-(Hydroxycarbamoyl)-butyl]-N-(3-pyridyl)-indol.

Beispiel 25: Eine Lösung von 5,0 g 3-(4-Carboxybutyl)-N-(3-pyridyl)-indol in 50 ml Methanol wird unter Stickstoff mit 0,25 g konzentrierter Schwefelsäure 2 h unter Rückfluss erhitzt. Das Reaktionsgemisch wird abgekühlt, eingedampft und zwischen 50 ml Aether und 50 ml eiskalter gesättigter Natriumhydrogencarbonatlösung verteilt. Die organische Phase wird abgetrennt, über Kaliumcarbonat getrocknet und eingedampft; man erhält 3-(4-Methoxycarbonylbutyl)-N-(3-pyridyl)-indol.

Beispiel 26: Eine Lösung von 6,1 g Aethanolamin und 2,94 g 3-(4-Carboxybutyl)-N-(3-pyridyl)-indol wird 3 h auf 170° erhitzt. Das überschüssige Aethanolamin wird durch Destillation unter vermindertem Druck entfernt; man erhält 3-[4-(4,5-Dihydrooxazol-2-yl)-butyl]-N-(3-pyridyl)-indol.

Beispiel 27: Eine Lösung von 6,0 g 3-(4-Carboxybutyl)-N-(3-pyridyl)-indol in 50 g Thionylchlorid wird 30 min unter Rückfluss erhitzt, abgekühlt und mit 50 ml Toluol versetzt. Die Lösungsmittel werden abgedampft und der Rückstand in 50 ml trockenem Methylenchlorid bei 0° wieder aufgelöst. Man leitet gasförmigen Ammoniak 30 min durch die Lösung. Nach dem Abdampfen des Lösungsmittels erhält man 3-(4-Carbamoylbutyl)-N-(3-pyridyl)-indol.

Entsprechend werden unter Verwendung von Methylamin bzw. Dimethylamin 3-[4-(N-Methylcarbamoyl)-butyl]-N-(3-pyridyl)-indol und 3-[4-(N,N-Dimethylcarbamoyl)-butyl]-N-(3-pyridyl)-indol hergestellt.

Beispiel 28: Eine Lösung von 0,75 g 5-Brom-N-(3-pyridyl)-indol und 0,5 g Acrylsäureäthylester in 20 ml Triäthylamin wird 48 h mit 10,8 mg Palladiumacetat und 30 mg Tri-o-Tolylphosphin unter Rückfluss erhitzt. Das Reaktionsgemisch wird abgekühlt, eingedampft und auf 20 g Kieselgel mit Diäthyläther chromatographiert, wobei man

5-(2-Aethoxycarbonyläthenyl)-N-(3-pyridyl)-indol erhält. Es wird durch 15-stündiges Erhitzen unter Rückfluss in 30 ml Aethanol mit 5 ml 2 n Natronlauge verseift; man erhält eine Lösung, die zur Trockene eingedampft wird. Der Rückstand wird zwischen 20 ml Aether und 10 ml Wasser verteilt. Die wässerige Phase wird abgetrennt und auf pH 5 gebracht; der entstandene Feststoff wird abfiltriert; man erhält 5-(2-Carboxyäthenyl)-N-(3-pyridyl)-indol, das unter Zersetzung bei 180° schmilzt.

5-Brom-N-(3-pyridyl)-indol wird aus 5-Bromindol und 3-Brompyridin gemäss den vorhergehenden Beispielen hergestellt.

Beispiel 29: Eine Lösung von 4,55 g p-Hydroxybenzoesäure-äthylester in 50 ml trockenem Dimethylformamid wird mit 1,31 g einer 50 %igen Natriumhydrid-Dispersion in Mineralöl unter Stickstoff bei 0° behandelt, 30 min bei 0° und dann 15 min bei Raumtemperatur gerührt. Eine Lösung von 7,55 g 3-(Methylsulfonyloxymethyl)-N-(3-pyridyl)-indol in 10 ml trockenem Dimethylformamid wird im Verlauf von 5 min zugegeben und das Reaktionsgemisch 18 h auf 50° erwärmt. Das Reaktionsgemisch wird auf Eis gegossen, mit konzentrierter Schwefelsäure angesäuert und mit Diäthyläther extrahiert (4 x 40 ml). Die wässerige Phase wird auf pH 8 eingestellt und mit Diäthyläther extrahiert (3 x 100 ml). Die organischen Extrakte werden mit Wasser (4 x 50 ml) und Natriumchloridlösung (1 x 50 ml) gewaschen und über wasserfreiem Natriumsulfat getrocknet. Nach Eindampfen und Chromatographieren auf 100 g Kieselgel mit Diäthyläther als Elutionsmittel erhält man 3-[(p-Aethoxycarbonylphenoxy)-methyl]-N-(3-pyridyl)-indol.

Die Ausgangsverbindung wird wie folgt hergestellt:
Eine Lösung von 3,3 g 3-Hydroxymethyl-N-(3-pyridyl)-indol in 25 ml Pyridin wird auf +5° abgekühlt und mit 1,36 ml Methansulfonylchlorid tropfenweise versetzt. Nach 3 h wird das Reaktionsgemisch mit 75 ml Eiswasser verdünnt und mit 1,5 g Natriumhydrogencarbonat versetzt. Nach der Extraktion mit Methylenchlorid (3 x 25 ml) und dem Trocknen

der Extrakte mit Natriumsulfat erhält man 3-(Methylsulfonyloxymethyl)-N-(3-pyridyl)-indol, das direkt in der nächsten Stufe verwendet wird.

Beispiel 30: Eine Lösung von rohem 3-[(p-Aethoxycarbonylphenoxy)-methyl]-N-(3-pyridyl)-indol (6,5 g) in 60 ml Aethanol und 60 ml 1 n Natronlauge wird 3 h unter Rückfluss erhitzt. Das Aethanol wird abdestilliert, die wässerige Phase wird mit Aether gewaschen, mit konzentrierter Salzsäure auf pH 6 angesäuert, und der entstandene Feststoff wird abfiltriert; man erhält 3-[(p-Carboxyphenoxy)-methyl]-N-(3-pyridyl)-indol. Die Behandlung mit Chlorwasserstoff in Aethanol gibt 3-[(p-Carboxyphenoxy)-methyl]-N-(3-pyridyl)-indol-Hydrochlorid.

Beispiel 31: Analog zu den Methoden der vorhergehenden Beispiele werden weitere Verbindungen der Formel I hergestellt, in denen $R_2 = R_3 = H$, Ar = 3-Pyridyl, $R_4$ = A-B in Stellung 3 des Indolrings bedeuten und worin B Carboxy darstellt.

| Verbindung | $(R_1)_p$ | A |
|---|---|---|
| 1 | 5-Cl | $(CH_2)_5$ |
| 2 | 5-Cl | $CH_2$ |
| 3 | 5-F | $(CH_2)_4$ |
| 4 | 5,6-diCl | $(CH_2)_4$ |
| 5 | 5,6-Methylendioxy | $(CH_2)_5$ |
| 6 | 5-OH | $(CH_2)_4$ |
| 7 | 5-$SCH_3$ | $(CH_2)_3$ |
| 8 | H | $(CH_2)_9$ |
| 9 | H | $-CH_2C \equiv C$ |
| 10 | H | $CH_2CH_2OCH_2$ |
| 11 | H | p-$(CH_2CH_2S)$-$C_6H_4$ |
| 12 | H | p-$(CH_2CH_2)$-$C_6H_4$ |
| 13 | 5-OMe | $CH(CH_3)$ |

Beispiel 32: Es werden 10.000 Tabletten hergestellt, die je 10 mg Wirkstoff enthalten:

Zusammensetzung

| | |
|---|---|
| 3-(4-Carboxybutyl)-5-chlor-N-(3-pyridyl)-indol | 100,00 g |
| Milchzucker | 1.157,00 g |
| Maisstärke | 75,00 g |
| Polyäthylenglykol 6.000 | 75,00 g |
| Talkumpulver | 75,00 g |
| Magnesiumstearat | 18,00 g |
| Gereinigtes Wasser | quantum satis |

Verfahren

Alle pulverförmigen Bestandteile werden durch ein Sieb mit einer Maschenweite von 0,6 mm gesiebt. Dann werden der Wirkstoff, Milchzucker, Talkum, Magnesiumstearat und die Hälfte der Stärke in einem geeigneten Mischer vermischt. Die andere Hälfte der Stärke wird in 40 ml Wasser suspendiert und die Suspension zur siedenden Lösung des Polyäthylenglykols in 150 ml Wasser gegeben. Die gebildete Paste wird den Pulvern zugesetzt und gegebenenfalls unter Zugabe von mehr Wasser granuliert. Das Granulat wird über Nacht bei 35° getrocknet, durch ein Sieb mit 1,2 mm Maschenweite getrieben und zu Tabletten von 6,4 mm Durchmesser, welche eine Bruchrille aufweisen, gepresst.

Beispiel 33: Es werden 10.000 Kapseln hergestellt, die je 25 mg des Wirkstoffs enthalten:

Zusammensetzung

| | |
|---|---|
| 3-(4-Carboxybutyl)-N-(3-pyridyl)-indol | 250,0 g |
| Milchzucker | 1.650,0 g |
| Talkumpulver | 100,0 g |

Verfahren

Alle pulverförmigen Bestandteile werden durch ein Sieb mit einer Maschenweite von 0,6 mm gesiebt. Dann wird der Wirkstoff in einen geeigneten Mischer eingebracht und zuerst mit Talkum und dann mit

Milchzucker bis zur Homogenität vermischt. Kapseln Nr. 3 werden mit je 200 mg der erhaltenen Mischung unter Verwendung einer Kapselfüllmaschine gefüllt.

Entsprechend werden Tabletten und Kapseln hergestellt, die etwa 10 bis 100 mg anderer erfindungsgemässer Verbindungen enthalten, z.B. von jeder anderen in den Beispielen angegebenen Verbindung.

Beispiel 34: Eine Lösung von 1,7 g 7-Oxooktansäure in 20 ml Essigsäure wird erhitzt, so dass sie siedet, und 2,0 g N-Phenyl-N-(3-pyridyl)-hydrazin in 5 ml Essigsäure werden tropfenweise hinzugegeben. Das Reaktionsgemisch wird 15 h unter Rückfluss gekocht, dann das Lösungsmittel fast bis zur Trockene eingedampft. Der Rückstand wird auf 20 g Eis gegossen und der pH-Wert auf 6 eingestellt. Das entstandene Oel kristallisiert beim Stehen aus, und man erhält 3-(4-Carboxybutyl)-2-methyl-N-(3-pyridyl)-indol des Beispiels 21/3, Fp. 112-114°.

Die Ausgangsverbindung wird wie folgt hergestellt:
Durch Kondensation von Anilin mit 3-Brompyridin unter üblichen Bedingungen der Ullmann-Reaktion (wie z.B. in J. Chem. Soc. C 1970, 85 beschrieben) erhält man N-(3-Pyridyl)-anilin.

Eine wässerige Lösung von 6,7 g Natriumnitrit wird tropfenweise zu einer Lösung von 17,0 g N-(3-Pyridyl)-anilin in 100 ml 2N Salzsäure gegeben. Das Reaktionsgemisch wird 1 h gerührt, und dann der pH-Wert durch Zugabe von 50 % Natronlauge auf 8 eingestellt. Der erhaltene Feststoff wird gesammelt, mit Wasser gewaschen, getrocknet, und man erhält N-Nitroso-N-(3-pyridyl)-anilin.

Eine Lösung von 10 g N-Nitroso-N-(3-pyridyl)-anilin in 100 ml Essigsäure und 50 ml Wasser wird mit 50 g Zinkstaub bei 10° 15 min lang behandelt. Der Ansatz wird 3 h auf 80° erwärmt, abgekühlt, filtriert und eingedampft. Der Rückstand wird mit Wasser aufgenommen und der pH-Wert mit 50 %iger Natronlauge auf 10 eingestellt. Die

erhaltene trübe Lösung wird mit Chloroform extrahiert. Die Extrakte werden über Kaliumcarbonat getrocknet, eingedampft, und man erhält N-Phenyl-N-(3-pyridyl)-hydrazin.

Beispiel 35: Zu einer Lösung von 3,06 g 2-Methyl-3-(5-oxohexyl)-N-(3-pyridyl)-indol in 25 ml Tetrahydrofuran und 10 ml 2N Natronlauge, die bei 0° gerührt wird, werden 20 ml einer 5 % Natriumhypochlorit-Lösung tropfenweise hinzugegeben. Das Reaktionsgemisch wird 30 min auf 70° erhitzt, abgekühlt und dann Natriumbisulfit hinzugegeben, um überschüssiges Natriumhypochlorit zu zerstören. Das organische Lösungsmittel wird abgedampft und der pH-Wert durch Zugabe konzentrierter Schwefelsäure auf 6 eingestellt. Man filtriert den ausgefallenen Feststoff ab und erhält 3-(4-Carboxybutyl)-2-methyl-N-(3-pyridyl)-indol des Beispiels 21/3, Fp. 112-114°.

Die Ausgangsverbindung wird wie folgt hergestellt:
Eine Lösung von 1,56 g 2,8-Nonandion in 20 ml Essigsäure wird zum Sieden erhitzt, und 2,0 g N-Phenyl-N-(3-pyridyl)-hydrazin in 10 ml Essigsäure werden tropfenweise hinzugegeben. Das Reaktionsgemisch wird 15 h unter Rückfluss gekocht, dann das Lösungsmittel fast bis zur Trockene eingedampft. Der Rückstand wird auf 20 g Eis gegossen und der pH-Wert mit 50 %iger Natronlauge auf 8 eingestellt. Der erhaltene Feststoff wird abfiltriert, getrocknet, und man erhält 2-Methyl-3-(5-oxohexyl)-N-(3-pyridyl)-indol.

Beispiel 36: Eine Lösung von 0,32 g 3-Jod-N-(3-pyridyl)-indol in 20 ml Tetrahydrofuran wird auf -100° abgekühlt, und 0,63 ml 1,6M tert.-Butyllithium werden tropfenweise hinzugegeben. Das Reaktionsgemisch wird 5 min gerührt, dann werden 0,2 g des Natriumsalzes der 5-Brompentansäure, gelöst in 1 ml Hexamethylphosphorsäuretriamid (HMPT), gegeben. Man lässt das Reaktionsgemisch auf Raumtemperatur erwärmen und rührt 2 h lang. Das Lösungsmittel wird abgedampft, der Rückstand wieder in Wasser aufgelöst und der pH-Wert auf 6 eingestellt. Der Feststoff wird abfiltriert, getrocknet, und man erhält 3-(4-Carboxybutyl)-N-(3-pyridyl)-indol des Beispiels 3, Fp. 122-124°.

Die Ausgangsverbindung wird wie folgt hergestellt:
Eine Lösung von 0,2 g N-(3-Pyridyl)-indol in 10 ml Methylenchlorid wird mit 0,25 g Jod bei 0° behandelt. Man lässt den Ansatz innerhalb von 1 h auf Raumtemperatur erwärmen, dampft ihn ein und erhält 3-Jod-N-(3-pyridyl)-indol-Hydrojodid. Man erhält die freie Base dadurch, dass man das Hydrojodid zu einer äquimolaren Menge Triäthylamin in Aether gibt, das entstandene Triäthylammoniumjodid abfiltriert und zur Trockene eindampft; so erhält man 3-Jod-N-(3-pyridyl)-indol.

Beispiel 37:

a) Ein Gemisch aus 0,30 g N-Acetyl-2-(3-carboxypropyl)-N-(3-pyridyl)-anilin und 0,6 g Kalium-tert.-butoxid in 10 ml trockenem Dekahydronaphthalin wird 3 h unter Rückfluss gekocht, dann abgekühlt und die Reaktion durch Zugabe von 10 ml Wasser beendet. Die wässerige Phase wird abgetrennt, mit 5 ml Aether gewaschen und mit konzentrierter Schwefelsäure auf pH 6 eingestellt. Das entstandene Oel kristallisiert aus, und man erhält 3-(2-Carboxyäthyl)-2-methyl-N-(3-pyridyl)-indol.

Die Ausgangsverbindung wird wie folgt hergestellt:
Eine Lösung von 10,0 g 2,3,4,5-Tetrahydro-1H-[1]-benzazepin-2-on in 20 ml 3-Brompyridin wird mit 6,0 g Kupferbronze 12 h unter Rückfluss gekocht. Die unlöslichen Bestandteile werden durch Filtration abgetrennt, und die Lösung wird zu einer gummiartigen Masse eingedampft, die über 200 g Kieselgel mit Aether als Elutionsmittel chromatographiert wird. Das Brompyridin wird mit der Lösungsmittelfront eluiert. Als zweite Substanz wird nicht umgesetztes Ausgangslactam eluiert, worauf das gewünschte N-(3-Pyridyl)-2,3,4,5-tetrahydro-1H-[1]-benzazepin-2-on folgt.

Eine Lösung von 5,0 g N-(3-Pyridyl)-2,3,4,5-tetrahydro-1H-[1]-benzazepin-2-on in 50 ml einer Mischung aus 25 ml Aethanol und 25 ml 50 % Natronlauge wird 10 h unter Rückfluss gekocht, dann abgekühlt und eingedampft. Der Rückstand wird in 10 ml Wasser gelöst, mit

10 ml Aether gewaschen und mit konzentrierter Schwefelsäure auf pH 6 gebracht. Das entstandene Oel kristallisiert beim Stehen aus, und man erhält o-(3-Carboxypropyl)-N-(3-pyridyl)-anilin.

Eine Lösung von 1,0 g o-(3-Carboxypropyl)-N-(3-pyridyl)-anilin in 10 ml Acetanhydrid wird 1 h unter Rückfluss gekocht, dann abgekühlt und eingedampft. Der Rückstand wird in Wasser gelöst und auf pH 6 eingestellt. Das erhaltene Oel wird durch Dekantieren abgetrennt und in Essigsäureäthylester erneut gelöst. Diese Lösung wird über Natriumsulfat getrocknet, eingedampft, und man erhält N-Acetyl-2-(3-carboxypropyl)-N-(3-pyridyl)-anilin.

b) Auf ähnliche Weise wird das 3-(4-Carboxybutyl)-2-methyl-N-(3-pyridyl)-indol des Beispiels 21/3 hergestellt, wobei man von 1,3,4,5,6,7-Hexahydro-1-benzazonin-2-on [Ann. Chem. 586, 30 (1954)] ausgeht.

Beispiel 38: Eine Lösung von 0,5 g o-(1-Formyl-4-carboxybutyl)-N-(3-pyridyl)-anilin in 10 ml Toluol wird zusammen mit 50 mg p-Toluolsulfonsäure und 0,5 g pulverförmigem Molekularsieb auf 90° erhitzt. Nach 4 h wird das Reaktionsgemisch abgekühlt und eingedampft. Der Rückstand wird in 5 ml 2N Salzsäure gelöst, dann der pH-Wert auf 7 eingestellt. Das erhaltene Oel wird mit Chloroform extrahiert und dieser Extrakt über Natriumsulfat getrocknet. Man verdampft das Lösungsmittel und erhält ein Oel, das beim Stehen auskristallisiert; man erhält 3-(3-Carboxypropyl)-N-(3-pyridyl)-indol des Beispiels 21/1, Fp. 134-135°.

Die Ausgangsverbindung wird wie folgt hergestellt:
Durch eine Ullmann-Kondensation mit o-Bromanilin und 3-Brompyridin erhält man o-Brom-N-(3-pyridyl)-anilin.

Zu einer Lösung von 2,4 g o-Brom-N-(3-pyridyl)-anilin in 50 ml trockenem Tetrahydrofuran bei -78° unter Stickstoff werden 8 ml 2,5M n-Butyllithium tropfenweise hinzugegeben. Nach 90 min wird die Lösung in ein Gemisch, bestehend aus 1,25 g Kupfer(I)bromid-Dimethylsulfid-Komplex in 50 ml Tetrahydrofuran und gehalten bei -40°, überführt. Das Reaktionsgemisch wird 30 min bei -40° gerührt, dann werden 0,48 g Cyclohexenon tropfenweise hinzugegeben. Nach 1 h werden 0,55 g Chlortrimethylsilan tropfenweise hinzugefügt, worauf man den Ansatz auf Raumtemperatur erwärmen lässt. Das Reaktionsgemisch wird 3 h bei Raumtemperatur gerührt und dann die Reaktion durch Zugabe von 20 ml Natriumhydrogencarbonat-Lösung abgebrochen. Der Feststoff wird abfiltriert. Die organische Phase wird abgetrennt, mit Kochsalzlösung gewaschen, kurz über trockenem Natriumsulfat getrocknet und eingedampft. Das erhaltene Oel, gelöst in Aether, wird schnell über 10 g Aluminiumoxid filtriert, wobei man rohes N-(3-Pyridyl)-o-(1-trimethylsiloxycyclohex-1-en-3-yl)-anilin erhält, das ohne Reinigung weiterverwendet wird.

Eine Lösung von 0,67 g N-(3-Pyridyl)-o-(1-trimethylsiloxycyclohex-1-en-3-yl)-anilin in 1,0 ml trockenem Dioxan wird langsam zu einer Lösung von 0,85 g Natriumperjodat und 0,1 g Osmiumtetroxid in 10 ml 30 % wässerigem Dioxan gegeben. Das Reaktionsgemisch wird 90 min bei 23° gerührt, dann zur Trockene eingedampft. Der Rückstand wird in 10 ml 1N Salzsäure aufgenommen, mit 10 ml Aether gewaschen und dann der pH-Wert auf 7 eingestellt. Das erhaltene Oel wird mit Chloroform extrahiert. Die Extrakte werden mit Wasser gewaschen, über Natriumsulfat getrocknet, eingedampft und man erhält o-(1-Formyl-4-carboxybutyl)-N-(3-pyridyl)-anilin als Oel.

Beispiel 39: Eine Lösung von 0,21 g 3-Methyl-N-(3-pyridyl)-indol in 2,0 ml Tetrahydrofuran wird langsam zu einer Lösung von Lithiumdiisopropylamid, hergestellt aus 0,11 g Diisopropylamin und 0,42 ml n-Butyllithium (0,24 m), in 15 ml Tetrahydrofuran bei -50° zugegeben. Man hält 1 h bei -50° und gibt dann tropfenweise eine Lösung von 0,2 g des Natriumsalzes der 4-Brombutansäure in 1,0 ml HMPT hinzu. Das Reaktionsgemisch wird auf 25° erwärmt und 5 h gerührt.

Man verdampft das Lösungsmittel, löst den Rückstand in Wasser und stellt den pH-Wert auf 6 ein. Der Feststoff wird abfiltriert, getrocknet, und man erhält das 3-(4-Carboxybutyl)-N-(3-pyridyl)-indol des Beispiels 3.

Patentansprüche für die Vertragsstaaten BE, CH, LI, DE, FR, GB, IT, LU, NL, SE

1. N-Pyridylindole der Formel I

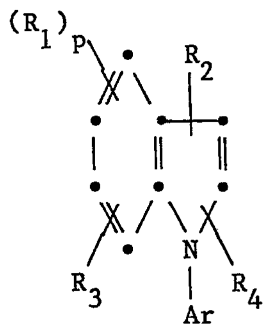

(I) ,

worin Ar für unsubstituiertes oder durch Niederalkyl substituiertes 3- oder 4-Pyridyl steht, die Reste $R_1$ unabhängig voneinander Wasserstoff, Halogen, Trifluormethyl, Niederalkyl, Hydroxy, acyliertes oder veräthertes Hydroxy oder Niederalkyl-(thio, -sulfinyl oder -sulfonyl) bedeuten, oder zwei an benachbarten Kohlenstoffatomen sitzende Reste $R_1$ Alkylendioxy darstellen, p für 1 oder 2 steht, $R_2$ Wasserstoff oder Niederalkyl bedeutet, einer der Reste $R_3$ und $R_4$ für Wasserstoff steht und der jeweils andere die Gruppe A-B bedeutet, in welcher

A für Alkylen mit 1 bis 12 Kohlenstoffatomen, Alkinylen oder Alkenylen mit jeweils 2 bis 12 Kohlenstoffatomen, Niederalkylenphenylen, Niederalkylen-(thio oder oxy)-niederalkylen, Niederalkylen-(thio oder oxy)-phenylen, Niederalkylenphenylen-nieder-(alkylen oder alkenylen) oder Alkadienylen mit 4 bis 12 Kohlenstoffatomen steht, und

B Carboxy, verestertes Carboxy, Carbamoyl, Mono- oder Di-niederalkylcarbamoyl, Cyan, Hydroxycarbamoyl, 5-Tetrazolyl, unsubstituiertes oder durch Niederalkyl substituiertes 4,5-Dihydro-2-oxazolyl bedeutet, ihre Pyridyl-N-oxide, und ihre Salze.

2. Verbindungen der Formel I gemäss Anspruch 1, worin Ar für unsubstituiertes oder durch Niederalkyl substituiertes 3- oder 4-Pyridyl steht, die Reste $R_1$ Wasserstoff, Halogen, Trifluormethyl,

Niederalkyl, Hydroxy, acyliertes oder veräthertes Hydroxy oder Niederalkyl-(thio, -sulfinyl oder -sulfonyl) bedeuten, oder zwei an benachbarten Kohlenstoffatomen sitzende Reste $R_1$ Alkylendioxy darstellen, p für 1 oder 2 steht, $R_2$ Wasserstoff oder Niederalkyl bedeutet, einer der Reste $R_3$ und $R_4$ für Wasserstoff steht und der jeweils andere die Gruppe A-B bedeutet, in welcher
A für Alkylen mit 1 bis 12 Kohlenstoffatomen, Alkinylen oder Alkenylen mit jeweils 2 bis 12 Kohlenstoffatomen, Niederalkylenphenylen, Niederalkylen-(thio oder oxy)-niederalkylen, Niederalkylen-(thio oder oxy)-phenylen, Niederalkylenphenylen-nieder-(alkylen oder alkenylen) oder Alkadienylen mit 4 bis 12 Kohlenstoffatomen steht, und
B Carboxy, verestertes Carboxy, Carbamoyl, Mono- oder Di-niederalkylcarbamoyl, Cyan, Hydroxycarbamoyl oder 5-Tetrazolyl bedeutet, ihre Pyridyl-N-oxide, und ihre Salze.

3. Verbindungen der Formel I gemäss Anspruch 1, worin Ar für 3-Pyridyl steht, $R_1$ Wasserstoff, Halogen, Trifluormethyl, Niederalkyl, Niederalkoxy, Niederalkylthio, Hydroxy oder Niederalkanoyloxy bedeutet, p für 1 steht, $R_2$ Wasserstoff oder Niederalkyl ist, $R_3$ Wasserstoff bedeutet und $R_4$ die Gruppe A-B darstellt, in der A die oben angegebene Bedeutung hat und B Carboxy, Niederalkoxycarbonyl, Carbamoyl, Cyan, Hydroxycarbamoyl oder 5-Tetrazolyl bedeutet; und ihre Salze.

0126401

4. Verbindungen der Formel II gemäss Anspruch 1,

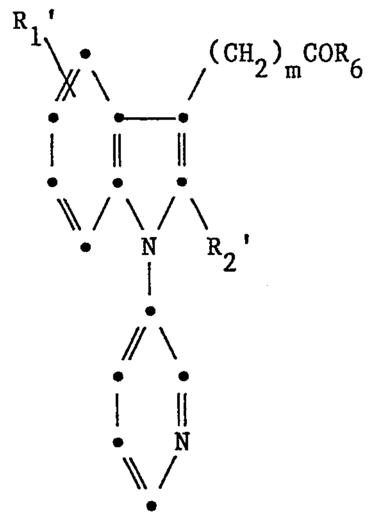

(II),

worin $R_1$' Wasserstoff, Niederalkyl, Halogen, Trifluormethyl, Hydroxy, Niederalkylthio oder Niederalkoxy bedeutet, $R_2$' Wasserstoff oder Niederalkyl ist, m für eine ganze Zahl von 1 bis 10 steht und $R_6$ Hydroxy, Niederalkoxy oder Amino bedeutet; und ihre pharmazeutisch verwendbaren Salze.

5. Verbindungen der Formel II gemäss Anspruch 4, worin $R_1$' Wasserstoff oder Chlor bedeutet, $R_2$' Wasserstoff ist, m für 4 oder 5 steht und $R_6$ Hydroxy bedeutet; und ihre pharmazeutisch verwendbaren Salze.

6. Verbindungen der Formel III gemäss Anspruch 1

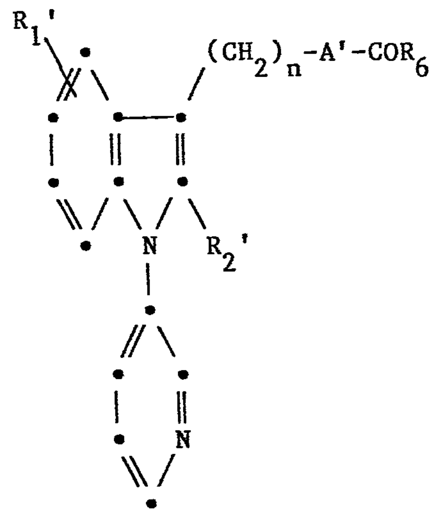

(III),

worin $R_1$' Wasserstoff, Niederalkyl, Halogen, Trifluormethyl, Hydroxy, Niederalkylthio oder Niederälkoxy bedeutet, $R_2$' Wasserstoff oder Niederalkyl ist, $R_6$ Hydroxy, Niederalkoxy oder Amino bedeutet, n für eine ganze Zahl von 1 bis 4 steht und A' (Thio oder Oxy)-alkylen mit 1 bis 4 Kohlenstoffatomen, (Thio- oder Oxy)-1,4-phenylen, 1,4-Phenylen, Aethenylen oder Niederalkyl-substituiertes Aethenylen bedeutet; und ihre pharmazeutisch verwendbaren Salze.

7. 3-(4-Carboxybutyl)-N-(3-pyridyl)-indol und pharmazeutisch verwendbare Salze davon gemäss Anspruch 1.

8. 3-(2-Carboxyäthyl)-N-(3-pyridyl)-indol und pharmazeutisch verwendbare Salze davon gemäss Anspruch 1.

9. (E)-3-(4-Carboxypent-3-enyl)-N-(3-pyridyl)-indol und pharmazeutisch verwendbare Salze davon gemäss Anspruch 1.

10. (Z)-3-(4-Carboxypent-3-enyl)-N-(3-pyridyl)-indol und pharmazeutisch verwendbare Salze davon gemäss Anspruch 1.

11. 3-(2-Carboxyprop-1-enyl)-N-(3-pyridyl)-indol und pharmazeutisch verwendbare Salze davon gemäss Anspruch 1.

12. 5-Brom-3-(4-carboxybutyl)-N-(3-pyridyl)-indol und pharmazeutisch verwendbare Salze davon gemäss Anspruch 1.

13. 3-(4-Carboxybutyl)-2-methyl-N-(3-pyridyl)-indol und pharmazeutisch verwendbare Salze davon gemäss Anspruch 1.

14. 3-(4-Carboxybutyl)-7-methyl-N-(3-pyridyl)-indol und pharmazeutisch verwendbare Salze davon gemäss Anspruch 1.

15. 3-(4-Carboxybutyl)-5-chlor-N-(3-pyridyl)-indol und pharmazeutisch verwendbare Salze davon gemäss Anspruch 1.

16. 3-(4-Carboxybutyl)-5-methyl-N-(3-pyridyl)-indol und pharmazeutisch verwendbare Salze davon gemäss Anspruch 1.

17. 3-(5-Carboxypentyl)-N-(3-pyridyl)-indol und pharmazeutisch verwendbare Salze davon gemäss Anspruch 1.

18. Eine Verbindung gemäss den Ansprüchen 1 oder 2 und ihre pharmazeutisch verwendbaren Salze zur Anwendung in einem Verfahren zur therapeutischen Behandlung des menschlichen oder tierischen Körpers.

19. Pharmazeutische Präparate enthaltend Verbindungen der Formel I gemäss den Ansprüchen 1 oder 2 oder pharmazeutisch verwendbare Salze von solchen Verbindungen.

20. Verwendung von Verbindungen der Formel I gemäss den Ansprüchen 1 oder 2 und von pharmazeutisch verwendbaren Salzen solcher Verbindungen.

21. Verbindungen der Formel I gemäss den Ansprüchen 1 oder 2 als Thromboxan-Synthetase-Hemmer.

22. Verfahren zur Herstellung von Verbindungen der Formel I, dadurch gekennzeichnet, dass man

1) eine Verbindung der Formel IV

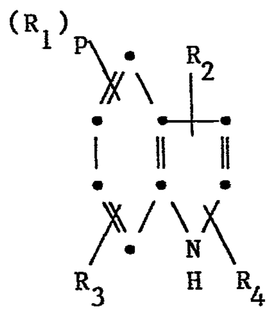

(IV),

worin $R_1$, $R_2$, $R_3$, $R_4$ und p die unter Formel I angegebene Bedeutung haben, mit einer Verbindung der Formel

Ar-X ,

worin Ar die unter Formel I angegebene Bedeutung hat und X für reaktives verestertes Hydroxy steht, umsetzt, oder

2) eine Verbindung der Formel V

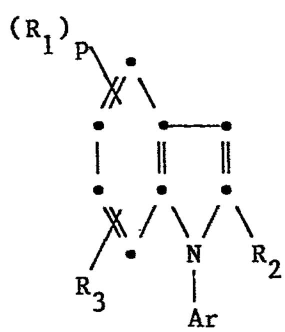

(V)

oder ein 3-Halogen-Derivat davon, worin Ar, $R_1$, p und $R_2$ die unter Formel I angegebene Bedeutung haben und $R_3$ Wasserstoff ist, mit einem reaktiven funktionellen Derivat einer Verbindung der Formel VI

HO - A - B          (VI),

worin A und B die unter Formel I angegebene Bedeutung haben, umsetzt, oder

3) eine Verbindung der Formel VII

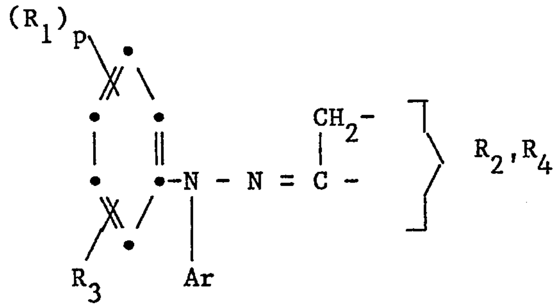

(VII),

die einen Rest $R_2$ und einen Rest $R_4$ enthält und worin $R_1$, $R_2$, $R_3$, $R_4$, p und Ar die unter Formel I angegebene Bedeutung haben, ringschliesst, oder

4) einer Verbindung der Formel VIII

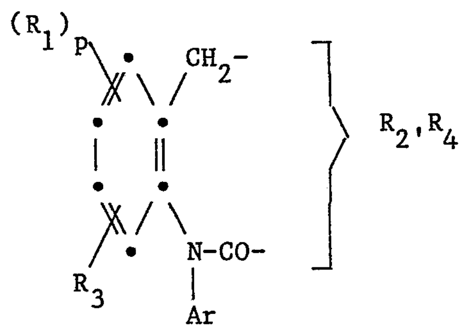

(VIII),

die eine Gruppe $R_2$ und eine Gruppe $R_4$ enthält und worin $R_1$, $R_2$, $R_3$, $R_4$, p und Ar die unter Formel I angegebene Bedeutung haben, cyclisiert oder

5) eine Verbindung der Formel IX

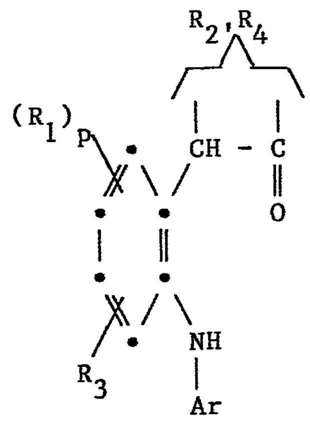

(IX),

die einen Rest $R_2$ und einen Rest $R_4$ enthält und worin $R_1$, $R_2$, $R_3$, $R_4$, p und Ar die unter Formel I angegebene Bedeutung haben, cyclisiert, oder

6) eine Verbindung der Formel Ia

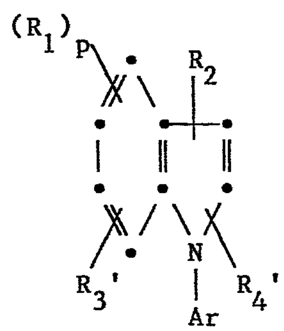

(Ia),

worin Ar, $R_1$, p und $R_2$ die unter Formel I angegebene Bedeutung haben, einer der beiden Reste $R_3'$ und $R_4'$ Wasserstoff und der jeweils andere die Gruppe A-C bedeutet, in der C eine von B verschiedene und in B überführbare Gruppe bedeutet, in eine Verbindung der Formel I umwandelt, gegebenenfalls unter Ausdehnung der Kette A innerhalb ihrer Definition, oder

7) eine Verbindung der Formel X

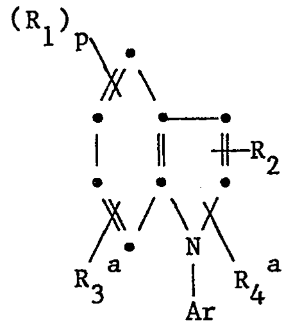

(X),

worin Ar, $R_1$, p und $R_2$ die unter Formel I angegebene Bedeutung haben, einer der beiden Reste $R_3^a$ und $R_4^a$ Wasserstoff bedeutet und der jeweils andere für Formyl oder Niederalkanoyl steht, mit einer Verbindung der Formel XI

$$R_5 - A'' - B \qquad (XI),$$

worin A" die unter Formel I für A definierte Bedeutung hat, jedoch die Kettenlänge um ein Kohlenstoffatom verkürzt ist, und $R_5$ einen Dialkylphosphono- oder Triarylphosphonium-Rest bedeutet; und, falls gewünscht, im erhaltenen Produkt der Formel I die Doppelbindung, welche direkt am Indolring sitzt, reduziert, oder

8) eine Verbindung der Formel XII

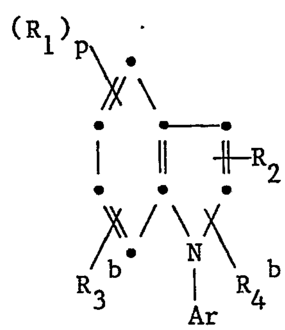

(XII),

worin Ar, $R_1$, p und $R_2$ die unter Formel I angegebene Bedeutung haben, einer der Reste $R_3^b$ und $R_4^b$ Wasserstoff bedeutet und der jeweils andere für reaktives verestertes Hydroxy steht, mit einer Verbindung der Formel XIII

$CH_2=CH-A'''-B$ (XIII),

worin A''' die unter Formel I für A definierte Bedeutung hat, worin jedoch die Kettenlänge um 2 Kohlenstoffatome verkürzt ist, oder worin A''' eine direkte Bindung bedeutet, umsetzt, und, wenn gewünscht, im erhaltenen Produkt der Formel I die Doppelbindung, die direkt am Indolring sitzt, reduziert; oder

9) eine Verbindung der Formel XVII

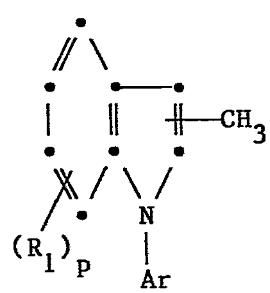

(XVII),

worin Ar, $R_1$ und p die unter Formel I angegebene Bedeutung haben, mit einem reaktiven funktionellen Derivat einer Verbindung VI'

HO - A'' - B (VI'),

worin A'' die unter Formel I für A angegebene Bedeutung hat, jedoch die Kettenlänge um ein Kohlenstoffatom verkürzt ist, und B die unter Formel I definierte Bedeutung hat, umsetzt; wobei störende reaktive Gruppen gegebenenfalls vorübergehend geschützt sind; und/oder, wenn erwünscht, eine erhaltene Verbindung der Formel I in eine andere Verbindung der Erfindung umwandelt, und/oder, wenn erwünscht, eine erhaltene freie Verbindung der Formel I in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

23. Die nach dem Verfahren gemäss Anspruch 22 erhältlichen Verbindungen.

FO 7.4 BL/bg*/eg*/am*

Patentansprüche für den Vertragsstaat AT

1. Verfahren zur Herstellung von N-Pyridylindolen der Formel I

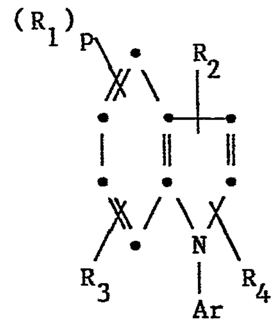

(I) ,

worin Ar für unsubstituiertes oder durch Niederalkyl substituiertes 3- oder 4-Pyridyl steht, die Reste $R_1$ unabhängig voneinander Wasserstoff, Halogen, Trifluormethyl, Niederalkyl, Hydroxy, acyliertes oder veräthertes Hydroxy oder Niederalkyl-(thio, -sulfinyl oder -sulfonyl) bedeuten, oder zwei an benachbarten Kohlenstoffatomen sitzende Reste $R_1$ Alkylendioxy darstellen, p für 1 oder 2 steht, $R_2$ Wasserstoff oder Niederalkyl bedeutet, einer der Reste $R_3$ und $R_4$ für Wasserstoff steht und der jeweils andere die Gruppe A-B bedeutet, in welcher

A für Alkylen mit 1 bis 12 Kohlenstoffatomen, Alkinylen oder Alkenylen mit jeweils 2 bis 12 Kohlenstoffatomen, Niederalkylen-phenylen, Niederalkylen-(thio oder oxy)-niederalkylen, Niederalkylen-(thio oder oxy)-phenylen, Niederalkylenphenylen-nieder-(alkylen oder alkenylen) oder Alkadienylen mit 4 bis 12 Kohlenstoffatomen steht, und

B Carboxy, verestertes Carboxy, Carbamoyl, Mono- oder Di-niederalkylcarbamoyl, Cyan, Hydroxycarbamoyl, 5-Tetrazolyl, unsubstituiertes oder durch Niederalkyl substituiertes 4,5-Dihydro-2-oxazolyl bedeutet, ihren Pyridyl-N-oxiden, und ihren Salzen, dadurch gekennzeichnet, dass man

1) eine Verbindung der Formel IV

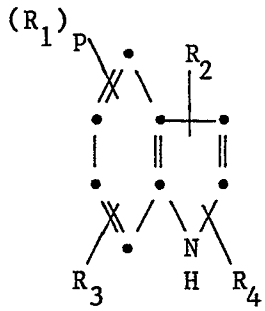

(IV),

worin $R_1$, $R_2$, $R_3$, $R_4$ und p die unter Formel I angegebene Bedeutung haben, mit einer Verbindung der Formel

Ar-X ,

worin Ar die unter Formel I angegebene Bedeutung hat und X für reaktives verestertes Hydroxy steht, umsetzt, oder

2) eine Verbindung der Formel V

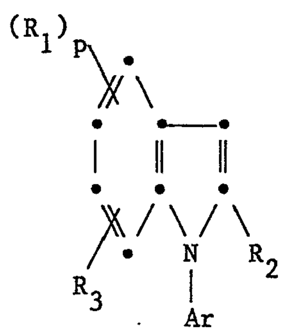

(V)

oder ein 3-Halogen-Derivat davon, worin Ar, $R_1$, p und $R_2$ die unter Formel I angegebene Bedeutung haben und $R_3$ Wasserstoff ist, mit einem reaktiven funktionellen Derivat einer Verbindung der Formel VI

HO - A - B (VI),

worin A und B die unter Formel I angegebene Bedeutung haben, umsetzt, oder

3) eine Verbindung der Formel VII

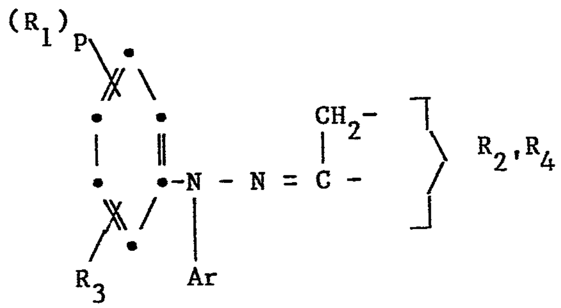

(VII),

die einen Rest $R_2$ und einen Rest $R_4$ enthält und worin $R_1$, $R_2$, $R_3$, $R_4$, p und Ar die unter Formel I angegebene Bedeutung haben, ringschliesst, oder

4) einer Verbindung der Formel VIII

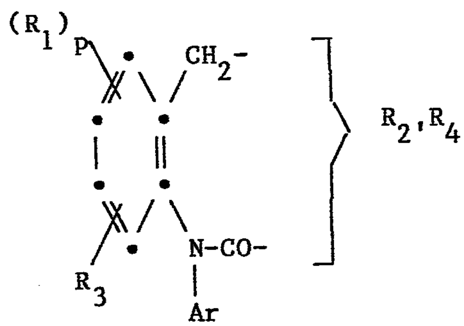

(VIII),

die eine Gruppe $R_2$ und eine Gruppe $R_4$ enthält und worin $R_1$, $R_2$, $R_3$, $R_4$, p und Ar die unter Formel I angegebene Bedeutung haben, cyclisiert oder

5) eine Verbindung der Formel IX

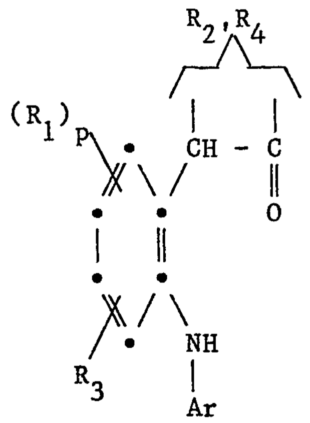

(IX),

die einen Rest $R_2$ und einen Rest $R_4$ enthält und worin $R_1$, $R_2$, $R_3$, $R_4$, p und Ar die unter Formel I angegebene Bedeutung haben, cyclisiert, oder

6) eine Verbindung der Formel Ia

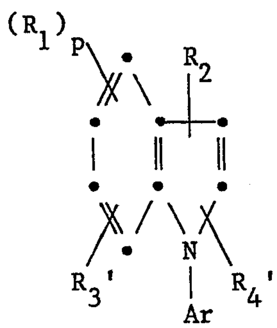

(Ia),

worin Ar, $R_1$, p und $R_2$ die unter Formel I angegebene Bedeutung haben, einer der beiden Reste $R_3'$ und $R_4'$ Wasserstoff und der jeweils andere die Gruppe A-C bedeutet, in der C eine von B verschiedene und in B überführbare Gruppe bedeutet, in eine Verbindung der Formel I umwandelt, gegebenenfalls unter Ausdehnung der Kette A innerhalb ihrer Definition, oder

7) eine Verbindung der Formel X

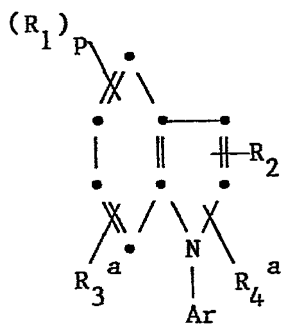

(X),

worin Ar, $R_1$, p und $R_2$ die unter Formel I angegebene Bedeutung haben, einer der beiden Reste $R_3^a$ und $R_4^a$ Wasserstoff bedeutet und der jeweils andere für Formyl oder Niederalkanoyl steht, mit einer Verbindung der Formel XI

$$R_5 - A'' - B \qquad (XI),$$

worin A" die unter Formel I für A definierte Bedeutung hat, jedoch die Kettenlänge um ein Kohlenstoffatom verkürzt ist, und $R_5$ einen Dialkylphosphono- oder Triarylphosphonium-Rest bedeutet; und, falls gewünscht, im erhaltenen Produkt der Formel I die Doppelbindung, welche direkt am Indolring sitzt, reduziert, oder

8) eine Verbindung der Formel XII

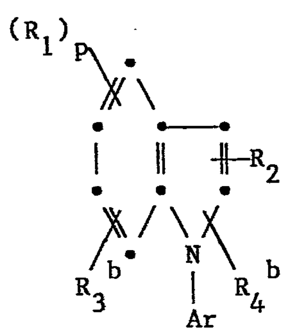

(XII),

worin Ar, $R_1$, p und $R_2$ die unter Formel I angegebene Bedeutung haben, einer der Reste $R_3^b$ und $R_4^b$ Wasserstoff bedeutet und der jeweils andere für reaktives verestertes Hydroxy steht, mit einer Verbindung der Formel XIII

$CH_2$=CH-A"'-B (XIII),

worin A"' die unter Formel I für A definierte Bedeutung hat, worin jedoch die Kettenlänge um 2 Kohlenstoffatome verkürzt ist, oder worin A"' eine direkte Bindung bedeutet, umsetzt, und, wenn gewünscht, im erhaltenen Produkt der Formel I die Doppelbindung, die direkt am Indolring sitzt, reduziert; oder

9) eine Verbindung der Formel XVII

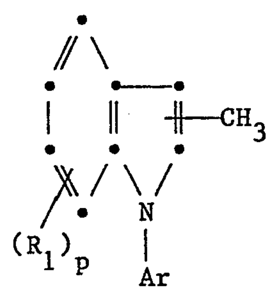

(XVII),

worin Ar, $R_1$ und p die unter Formel I angegebene Bedeutung haben, mit einem reaktiven funktionellen Derivat einer Verbindung VI'

HO - A" - B (VI'),

worin A" die unter Formel I für A angegebene Bedeutung hat, jedoch die Kettenlänge um ein Kohlenstoffatom verkürzt ist, und B die unter Formel I definierte Bedeutung hat, umsetzt; wobei störende reaktive Gruppen gegebenenfalls vorübergehend geschützt sind; und/oder, wenn erwünscht, eine erhaltene Verbindung der Formel I in eine andere Verbindung der Erfindung umwandelt, und/oder, wenn erwünscht, eine erhaltene freie Verbindung der Formel I in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

2. Verfahren gemäss Anspruch 1, dadurch gekennzeichnet, dass man Verbindungen der Formel I, worin Ar für 3-Pyridyl steht, $R_1$ Wasserstoff, Halogen, Trifluormethyl, Niederalkyl, Niederalkoxy, Niederalkylthio, Hydroxy oder Niederalkanoyloxy bedeutet, p für 1 steht, $R_2$ Wasserstoff oder Niederalkyl ist, $R_3$ Wasserstoff bedeutet und $R_4$ die Gruppe A-B darstellt, in der A die oben angegebene Bedeutung hat und B Carboxy, Niederalkoxycarbonyl, Carbamoyl, Cyan, Hydroxycarbamoyl oder 5-Tetrazolyl bedeutet; und ihre Salze herstellt.

3. Verfahren gemäss Anspruch 1, dadurch gekennzeichnet, dass man Verbindungen der Formel II

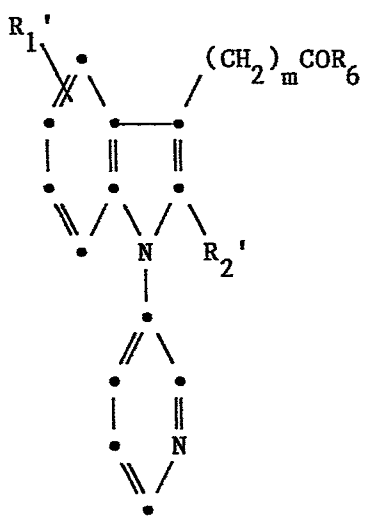

(II),

worin $R_1'$ Wasserstoff, Niederalkyl, Halogen, Trifluormethyl, Hydroxy, Niederalkylthio oder Niederalkoxy bedeutet, $R_2'$ Wasserstoff oder Niederalkyl ist, m für eine ganze Zahl von 1 bis 10 steht und $R_6$ Hydroxy, Niederalkoxy oder Amino bedeutet; und ihre pharmazeutisch verwendbaren Salze herstellt.

4. Verfahren gemäss Anspruch 3, dadurch gekennzeichnet, dass man Verbindungen der Formel II, worin $R_1'$ Wasserstoff oder Chlor bedeutet, $R_2'$ Wasserstoff ist, m für 4 oder 5 steht und $R_6$ Hydroxy bedeutet; und ihre pharmazeutisch verwendbaren Salze herstellt.

5. Verfahren gemäss Anspruch 1, dadurch gekennzeichnet, dass man Verbindungen der Formel III

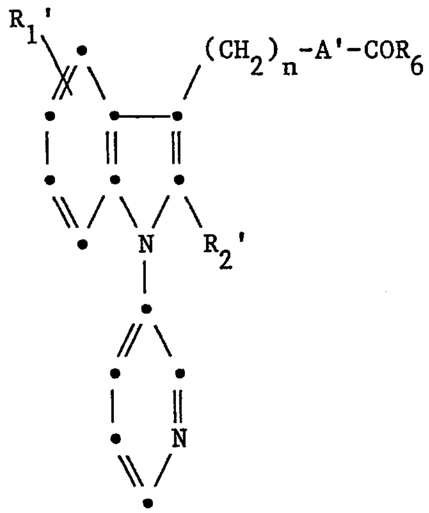

(III),

worin $R_1$' Wasserstoff, Niederalkyl, Halogen, Trifluormethyl, Hydroxy, Niederalkylthio oder Niederalkoxy bedeutet, $R_2$' Wasserstoff oder Niederalkyl ist, $R_6$ Hydroxy, Niederalkoxy oder Amino bedeutet, n für eine ganze Zahl von 1 bis 4 steht und A' (Thio oder Oxy)-alkylen mit 1 bis 4 Kohlenstoffatomen, (Thio- oder Oxy)-1,4-phenylen, 1,4-Phenylen, Aethenylen oder Niederalkyl-substituiertes Aethenylen bedeutet; und ihre pharmazeutisch verwendbaren Salze herstellt.

6. Verfahren gemäss Anspruch 1, dadurch gekennzeichnet, dass man 3-(4-Carboxybutyl)-N-(3-pyridyl)-indol und pharmazeutisch verwendbare Salze davon herstellt.

7. Verfahren gemäss Anspruch 1, dadurch gekennzeichnet, dass man 3-(2-Carboxyäthyl)-N-(3-pyridyl)-indol und pharmazeutisch verwendbare Salze davon herstellt.

8. Verfahren gemäss Anspruch 1, dadurch gekennzeichnet, dass man (E)-3-(4-Carboxypent-3-enyl)-N-(3-pyridyl)-indol und pharmazeutisch verwendbare Salze davon herstellt.

9. Verfahren gemäss Anspruch 1, dadurch gekennzeichnet, dass man (Z)-3-(4-Carboxypent-3-enyl)-N-(3-pyridyl)-indol und pharmazeutisch verwendbare Salze davon herstellt.

10. Verfahren gemäss Anspruch 1, dadurch gekennzeichnet, dass man 3-(2-Carboxyprop-1-enyl)-N-(3-pyridyl)-indol und pharmazeutisch verwendbare Salze davon herstellt.

11. Verfahren gemäss Anspruch 1, dadurch gekennzeichnet, dass man 5-Brom-3-(4-carboxybutyl)-N-(3-pyridyl)-indol und pharmazeutisch verwendbare Salze davon herstellt.

12. Verfahren gemäss Anspruch 1, dadurch gekennzeichnet, dass man 3-(4-Carboxybutyl)-2-methyl-N-(3-pyridyl)-indol und pharmazeutisch verwendbare Salze davon herstellt.

13. Verfahren gemäss Anspruch 1, dadurch gekennzeichnet, dass man 3-(4-Carboxybutyl)-7-methyl-N-(3-pyridyl)-indol und pharmazeutisch verwendbare Salze davon herstellt.

14. Verfahren gemäss Anspruch 1, dadurch gekennzeichnet, dass man 3-(4-Carboxybutyl)-5-chlor-N-(3-pyridyl)-indol und pharmazeutisch verwendbare Salze davon herstellt.

15. Verfahren gemäss Anspruch 1, dadurch gekennzeichnet, dass man 3-(4-Carboxybutyl)-5-methyl-N-(3-pyridyl)-indol und pharmazeutisch verwendbare Salze davon herstellt.

16. Verfahren gemäss Anspruch 1, dadurch gekennzeichnet, dass man 3-(5-Carboxypentyl)-N-(3-pyridyl)-indol und pharmazeutisch verwendbare Salze davon herstellt.

17. Verfahren zur Herstellung von Verbindungen der Formel I gemäss Anspruch 1, worin Ar für unsubstituiertes oder durch Niederalkyl substituiertes 3- oder 4-Pyridyl steht, die Reste $R_1$ Wasserstoff, Halogen, Trifluormethyl, Niederalkyl, Hydroxy, acyliertes oder

veräthertes Hydroxy oder Niederalkyl-(thio, -sulfinyl oder -sulfonyl) bedeuten, oder zwei an benachbarten Kohlenstoffatomen sitzende Reste $R_1$ Alkylendioxy darstellen, p für 1 oder 2 steht, $R_2$ Wasserstoff oder Niederalkyl bedeutet, einer der Reste $R_3$ und $R_4$ für Wasserstoff steht und der jeweils andere die Gruppe A-B bedeutet, in welcher

A für Alkylen mit 1 bis 12 Kohlenstoffatomen, Alkinylen oder Alkenylen mit jeweils 2 bis 12 Kohlenstoffatomen, Niederalkylenphenylen, Niederalkylen-(thio oder oxy)-niederalkylen, Niederalkylen-(thio oder oxy)-phenylen, Niederalkylenphenylen-nieder-(alkylen oder alkenylen) oder Alkadienylen mit 4 bis 12 Kohlenstoffatomen steht, und

B Carboxy, verestertes Carboxy, Carbamoyl, Mono- oder Di-niederalkylcarbamoyl, Cyan, Hydroxycarbamoyl oder 5-Tetrazolyl, bedeutet, ihre Pyridyl-N-oxide, und ihre Salze, dadurch gekennzeichnet, dass man

1) eine Verbindung der Formel IV

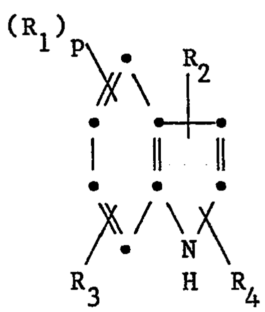

(IV),

worin $R_1$, $R_2$, $R_3$, $R_4$ und p die unter Formel I angegebene Bedeutung haben, mit einer Verbindung der Formel

Ar-X ,

worin Ar die unter Formel I angegebene Bedeutung hat und X für reaktives verestertes Hydroxy steht, umsetzt, oder

2) eine Verbindung der Formel V

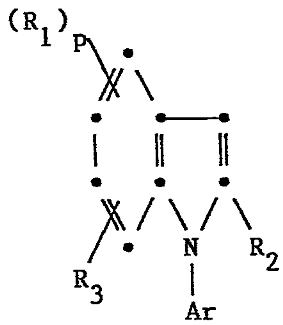

(V)

oder ein 3-Halogen-Derivat davon, worin Ar, $R_1$, p und $R_2$ die unter Formel I angegebene Bedeutung haben und $R_3$ Wasserstoff ist, mit einem reaktiven funktionellen Derivat einer Verbindung der Formel VI

$$HO - A - B \qquad (VI),$$

worin A und B die unter Formel I angegebene Bedeutung haben, umsetzt, wobei störende reaktive Gruppen gegebenenfalls vorübergehend geschützt sind, oder

3) eine Verbindung der Formel VII

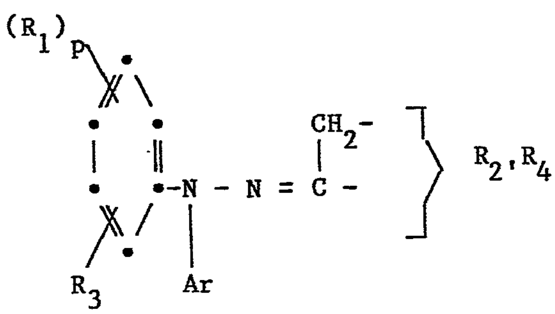

(VII),

die einen Rest $R_2$ und einen Rest $R_4$ enthält und worin $R_1$, $R_2$, $R_3$, $R_4$, p und Ar die unter Formel I angegebene Bedeutung haben, ringschliesst, oder

4) einer Verbindung der Formel VIII

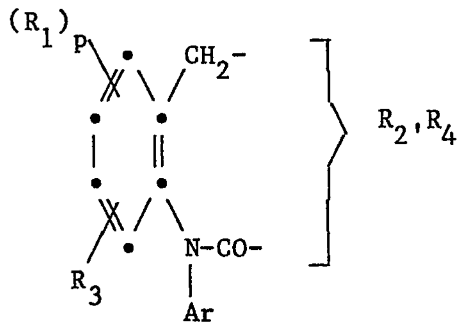

(VIII),

die eine Gruppe $R_2$ und eine Gruppe $R_4$ enthält und worin $R_1$, $R_2$, $R_3$, $R_4$, p und Ar die unter Formel I angegebene Bedeutung haben, cyclisiert oder

5) eine Verbindung der Formel IX

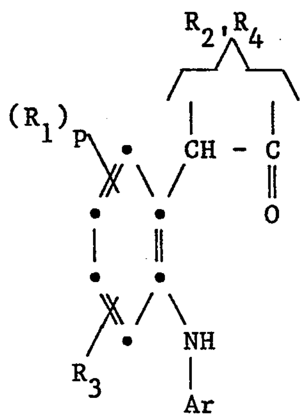

(IX),

die einen Rest $R_2$ und einen Rest $R_4$ enthält und worin $R_1$, $R_2$, $R_3$, $R_4$, p und Ar die unter Formel I angegebene Bedeutung haben, cyclisiert, oder

6) eine Verbindung der Formel Ia

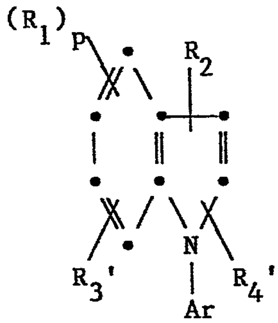

(Ia),

worin Ar, $R_1$, p und $R_2$ die unter Formel I angegebene Bedeutung haben, einer der beiden Reste $R_3'$ und $R_4'$ Wasserstoff und der jeweils andere die Gruppe A-C bedeutet, in der C eine von B verschiedene und in B überführbare Gruppe bedeutet, in eine Verbindung der Formel I umwandelt; oder

7) eine Verbindung der Formel X

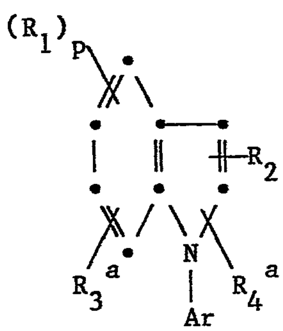

(X),

worin Ar, $R_1$, p und $R_2$ die unter Formel I angegebene Bedeutung haben, einer der beiden Reste $R_3^a$ und $R_4^a$ Wasserstoff bedeutet und der jeweils andere für Formyl oder Niederalkanoyl steht, mit einer Verbindung der Formel XI

$$R_5 - A'' - B \qquad (XI),$$

worin A" die unter Formel I für A definierte Bedeutung hat, jedoch die Kettenlänge um ein Kohlenstoffatom verkürzt ist, und $R_5$ einen Dialkylphosphono- oder Triarylphosphonium-Rest bedeutet; und, falls gewünscht, im erhaltenen Produkt die Doppelbindung, welche direkt am Indolring sitzt, reduziert, oder

8) eine Verbindung der Formel XII

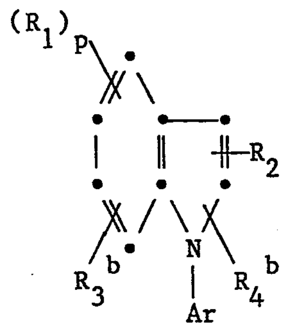

(XII),

worin Ar, $R_1$, p und $R_2$ die unter Formel I angegebene Bedeutung haben, einer der Reste $R_3^b$ und $R_4^b$ Wasserstoff bedeutet und der jeweils andere für reaktives verestertes Hydroxy steht, mit einer Verbindung der Formel XIII

$$CH_2=CH-A'''-B \qquad (XIII),$$

worin A"' die unter Formel I für A definierte Bedeutung hat, worin jedoch die Kettenlänge um 2 Kohlenstoffatome verkürzt ist, oder worin A"' eine direkte Bindung bedeutet, umsetzt, und, wenn gewünscht, im erhaltenen Produkt I die Doppelbindung, die direkt am Indolring sitzt, reduziert; und/oder, wenn erwünscht, eine gemäss dem oben beschriebenen Verfahren erhaltene Verbindung der Formel I in eine andere Verbindung der Formel I umwandelt, und/oder, wenn erwünscht, eine gemäss dem oben beschriebenen Verfahren erhaltene freie Verbindung der Formel I in ein Salz oder ein erhaltenes Salz in die freie Verbindung überführt, und/oder, wenn erwünscht, ein optisches oder geometrisches Isomeres, das angereichert ist, aus einer Mischung von Isomeren einer erhaltenen Verbindung der Formel I isoliert.

18. Verfahren gemäss Anspruch 17, dadurch gekennzeichnet, dass man 3-(4-Carboxybutyl)-N-(3-pyridyl)-indol und pharmazeutisch verwendbare Salze davon herstellt.

19. Verfahren gemäss Anspruch 17, dadurch gekennzeichnet, dass man 3-(4-Carboxybutyl)-5-chlor-N-(3-pyridyl)-indol und pharmazeutisch verwendbare Salze davon herstellt.

20. Verfahren zur Herstellung von pharmazeutischen Präparaten, dadurch gekennzeichnet, dass man eine nach dem Verfahren gemäss Anspruch 1 erhaltene Verbindung der Formel I oder ein pharmazeutisch verwendbares Salz einer solcher Verbindung mit einem pharmazeutisch verwendbaren Trägermaterial mischt.

FO 7.4 BL/bg*/eg*/am*

Europäisches Patentamt

## EUROPÄISCHER RECHERCHENBERICHT

0126401

Nummer der Anmeldung

EP 84 10 5449

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | AT-B- 299 938 (FABBRICA ITALIANA SINTETICI)<br>* Insgesamt *<br>--- | 1 | C 07 D 401/04<br>A 61 K 31/405 |
| Y | FR-A-2 171 937 (CLIN MIDY)<br>* Seiten 9,11,12 *<br>--- | 1 | |
| Y | AT-B- 323 154 (HURKA, W.)<br>* Insgesamt *<br>----- | | |
| | | | **RECHERCHIERTE SACHGEBIETE (Int. Cl. 3)** |
| | | | C 07 D 401/00<br>C 07 D 209/00 |

Der vorliegende Recherchenbericht wurde für alle Patentansprüche erstellt.

| Recherchenort | Abschlußdatum der Recherche | Prüfer |
|---|---|---|
| DEN HAAG | 16-08-1984 | MAISONNEUVE J.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82